(19) **Europäisches Patentamt** / **European Patent Office** / **Office européen des brevets**

(11) **EP 4 546 357 B1**

(12) **EUROPEAN PATENT SPECIFICATION**

(45) Date of publication and mention of the grant of the patent:
**15.07.2026 Bulletin 2026/29**

(21) Application number: **22949090.9**

(22) Date of filing: **23.11.2022**

(51) International Patent Classification (IPC):
**G16H 15/00** (2018.01)    **G16H 10/60** (2018.01)
**G06F 16/215** (2019.01)    **A61M 16/00** (2006.01)
**G16H 20/40** (2018.01)

(52) Cooperative Patent Classification (CPC):
**G16H 15/00; A61B 5/0205; G16H 10/60;**
**G16H 20/40; G16H 40/63; G16H 50/20;**
**G16H 50/70;** Y02A 90/10

(86) International application number:
**PCT/CN2022/133811**

(87) International publication number:
**WO 2024/001010 (04.01.2024 Gazette 2024/01)**

(54) **MECHANICAL VENTILATION TREATMENT DATA MANAGEMENT METHOD AND SYSTEM**

VERFAHREN UND SYSTEM ZUR VERWALTUNG VON DATEN EINER MECHANISCHEN BEATMUNGSBEHANDLUNG

PROCÉDÉ ET SYSTÈME DE GESTION DE DONNÉES DE TRAITEMENT DE VENTILATION MÉCANIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **01.07.2022 CN 202210769891**

(43) Date of publication of application:
**30.04.2025 Bulletin 2025/18**

(73) Proprietor: **Shanghai SVM Medical Technology Co., Ltd.**
**Shanghai 200233 (CN)**

(72) Inventors:
- **LONG, Yun**
  **Shanghai 200233 (CN)**
- **HUANG, Xiaobo**
  **Shanghai 200233 (CN)**
- **DU, Bin**
  **Shanghai 200233 (CN)**
- **LI, Yingchuan**
  **Shanghai 200233 (CN)**
- **SU, Longxiang**
  **Shanghai 200233 (CN)**
- **SHANG, You**
  **Shanghai 200233 (CN)**
- **PAN, Chun**
  **Shanghai 200233 (CN)**
- **LAN, Yunping**
  **Shanghai 200233 (CN)**
- **HE, Hongli**
  **Shanghai 200233 (CN)**
- **ZHAI, Qian**
  **Shanghai 200233 (CN)**
- **ZHANG, Jicheng**
  **Shanghai 200233 (CN)**
- **HE, Huaiwu**
  **Shanghai 200233 (CN)**
- **CHI, Yi**
  **Shanghai 200233 (CN)**
- **HUANG, Man**
  **Shanghai 200233 (CN)**
- **HAN, Xiaotong**
  **Shanghai 200233 (CN)**
- **SHAO, Huanzhang**
  **Shanghai 200233 (CN)**
- **ZHUANG, Yugang**
  **Shanghai 200233 (CN)**
- **SHANG, Xiuling**
  **Shanghai 200233 (CN)**

- **ZHANG, Song**
  **Shanghai 200233 (CN)**
- **LI, Xiantao**
  **Shanghai 200233 (CN)**
- **SHEN, Ning**
  **Shanghai 200233 (CN)**
- **HAO, Xiaoning**
  **Shanghai 200233 (CN)**
- **TANG, Zujun**
  **Shanghai 200233 (CN)**
- **LIU, Xianlong**
  **Shanghai 200233 (CN)**
- **CAI, Fuhong**
  **Shanghai 200233 (CN)**
- **ZHU, Bin**
  **Shanghai 200233 (CN)**

(74) Representative: **ABG Intellectual Property Law, S.L.**
**Avenida de Burgos, 16D**
**Edificio Euromor**
**28036 Madrid (ES)**

(56) References cited:
| | |
|---|---|
| **EP-B1- 2 249 700** | **CN-A- 105 147 278** |
| **CN-A- 108 648 786** | **CN-A- 109 431 491** |
| **CN-A- 110 189 822** | **CN-A- 113 577 476** |
| **CN-A- 113 577 476** | **CN-A- 115 116 575** |
| **US-A1- 2020 230 336** | **US-A1- 2021 074 415** |
| **US-B2- 10 874 811** | |

## Description

### TECHNICAL FIELD

The present application relates to the field of mechanical ventilation therapy data management and real-time analysis technologies, and particularly relates to a method and system of mechanical ventilation therapy data management.

### BACKGROUND

Mechanical ventilation therapy technology has been widely used in intensive care units (ICU, NICU, RICU, EICU, etc.), anesthesiology departments and other departments in hospitals all over the world. Every year, tens of millions of critically ill patients around the world use this treatment technology, if used improperly, it will cause complications such as lung injury, ventilator-associated pneumonia, arrhythmia, acute heart failure, etc., aggravate the original disease, and endanger the patients' lives.

In the context of a common mechanical ventilation therapy, the patient is connected with a ventilator and a multi-parameter monitor, doctors and nurses observe closely and analyze the continuous mechanical ventilation waveform and numerical data on the ventilator screen to determine the working status of the ventilator, to analyze the changes in the patient's respiratory mechanics, the disease evolution, and the state of patient-ventilator interactions, and at the same time to observe the changes in physiological parameters of heart rate, blood pressure, blood oxygen saturation, and body temperature. It is also necessary to observe the blood gas analysis, laboratory diagnosis, imaging and other data through the clinical information systems (HIS/CIS/EMR system) in order to fully understand the disease status and evolution trend, adjust ventilator parameters and treatment strategies, prevent and control various complications.

However, each ventilator generates up to hundreds of MBs (Mega Bytes) of waveform data in 24 hours, doctors and nurses frequently check back and forth on the screens of various devices every day to observe and analyze all kinds of data, and at the same time, they also need to understand the patient's medical condition in the past time every day, and retrieve the 24/48-hours mechanical ventilation data, physiological parameters and other data during treatment from the devices or the clinical information systems. However, the devices and the clinical information systems only store numerical data, lack important waveform data, and contain a large amount of interference data, as well as data that the device itself analyzes incorrectly, so that doctors and nurses need to spend a lot of time reading and analyzing the data segment by segment, and performing identification and judgment on the data manually. Obviously, this clinical application way of the data causes heavy work pressure and requires a large amount of manpower, however, ventilators from various manufacturers are unable to provide 24-hours mechanical ventilation data analysis reports, while there is a general shortage of doctors and nurses in ICUs in hospitals around the world. So far, this situation has not changed.

In addition, one hospitals are often equipped with dozens to hundreds of ventilators and multi-parameter monitors from different manufacturers, there are complex problems such as different data communication protocols and data formats for similar devices, different data communication protocols and data formats for different kinds of devices, and clinical information systems are also from different manufacturers, which increase the difficulty of mechanical ventilation therapy data processing. Over the past decades, ventilator technology has developed continuously, but the clinical application way of the mechanical ventilation therapy data has not progressed, and there are significant shortcomings.

US 2020/230336 A1 discloses systems and methods for clinician decision support during mechanical ventilation of a patient.

CN 113577476 A discloses a medical data analysis system based on respirator information collection.

US 2021/074415 A1 relates to a system and method for sharing data on a medical cloud platform based on third-party business.

US 10874811 B2 describes a system that automatically detects patient-ventilator asynchrony and trends in patient-ventilator asynchrony.

EP2249700 B1 discloses an apparatus for predicting patient respiratory stability.

### SUMMARY

In view of the above analysis, embodiments of the present application aim at providing a method and system of mechanical ventilation therapy data management, to solve the problems of many difficulties caused by huge heterogeneous data to clinical application, poor medical quality of mechanical ventilation therapy, and low working efficiency of data processing by doctors and nurses.

In one aspect, the present application discloses a method of mechanical ventilation therapy data management, as defined in claim 1.

Further, in the physiological parameters data, the waveform data comprises: electrocardiogram waveform data, respiratory waveform data, photoplethysmogram waveform data and invasive arterial pressure waveform data; the numerical data comprises: heart rate, blood pressure, blood oxygen saturation and body temperature;

in the data labeling process for the physiological parameters data,
the shapes of the labeled electrocardiogram waveform data comprise: P wave, QRS complex, T wave, ST segment, PR interval, RR interval and QT interval of each ECG cycle waveform;
the shapes of the labeled respiratory waveform data comprise: peaks and valleys of each respiratory cycle waveform;
the shapes of the labeled photoplethysmogram waveform data comprise: peaks and valleys of each pulse cycle waveform;
the shapes of the labeled invasive arterial pressure waveform data comprise: peaks and valleys of each arterial systolic-diastolic cycle waveform;
the labeled abnormal events comprise: tachycardia, bradycardia, arrhythmia, cardiac arrest, atrial flutter, atrial fibrillation, ventricular flutter, ventricular fibrillation, atrioventricular block, ST-segment elevation/depression, high/low heart rate, high/low blood pressure, high/low body temperature and low blood oxygen saturation.

Further, in the blood gas analysis data, the numerical data comprises: partial pressure of oxygen and carbon dioxide, total carbon dioxide, potassium ion concentration, sodium ion concentration, bicarbonate ion concentration and pH value;

in the data labeling process for the blood gas analysis data,
the labeled abnormal events comprise: high/low partial pressure of oxygen, high/low partial pressure of carbon dioxide, high/low total carbon dioxide, high/low potassium ion concentration, high/low sodium ion concentration and high/low pH value.

Further, in the laboratory diagnostic data, the numerical data comprises: white blood cell count, red blood cell count, platelet count, hemoglobin, hematocrit, blood glucose, prothrombin time and activated partial thrombin time;

in the data labeling process for the laboratory diagnostic data,
the labeled abnormal events comprise: high/low white blood cell count, high/low red blood cell count, high/low hemoglobin, high/low hematocrit, high/low blood glucose, high/low platelet count, long/short prothrombin time, and long/short activated partial thrombin time.

Further, the reporting framework of the mechanical ventilation therapy data analysis report comprises a report overview page and a report analysis page; wherein, the report overview page is divided into a basic information area, a mechanical ventilation data area, and an analysis conclusion area;

the basic information area comprises at least the patient information and key time point information; and further comprises the following options: the clinical diagnosis information, the ID of the device for outputting the mechanical ventilation therapy data as well as details regarding mechanical ventilation mode and settings;
the mechanical ventilation data area comprises at least respiratory analysis results, patient-ventilator asynchronous events analysis results, airway resistance change analysis results, compliance change analysis results, and lung injury early warning analysis results; each of the analysis results of the mechanical ventilation data area is obtained based on the mechanical ventilation data and its data labeling; the respiratory analysis results comprise a rapid shallow breathing index;
the analysis conclusion area is configured to display the analysis descriptions and conclusions of the mechanical ventilation therapy data;
the report analysis page is configured to display a detailed analysis report of one or more mechanical ventilation therapy data separately in graphical form.

Further, the report analysis page comprises a trend graph page; the trend graph page comprises:

trend analysis areas of pressure, volume, and ventilation volume of the mechanical ventilation data;
a trend analysis area of a respiratory frequency;
a trend analysis area of an inhaled oxygen concentration; and,
a trend analysis description and conclusion area, configured to synthesize the trend direction of each trend analysis area to provide trend analysis descriptions and the corresponding conclusions;
the content of each trend analysis area in the trend graph page is obtained based on the mechanical ventilation data and its data labeling.

Further, the report analysis page further comprises a data page;
the data page comprises at least:

a mechanical ventilation setting parameters data sub-page, comprising: a mechanical ventilation setting parameters record sheet, ventilation modes of mechanical ventilation and a change trend graph of setting parameters;
a mechanical ventilation monitoring parameters data sub-page, comprising a mechanical ventilation monitoring parameters record sheet;
the contents of the mechanical ventilation setting parameters data sub-page and the mechanical ventilation monitoring parameters data sub-page are obtained based on the mechanical ventilation data and its data labeling;
the data page further comprises the following optional data sub-pages:

a 24-hour physiological parameters data sub-page, comprising a 24-hour vital signs monitoring record sheet and a heart rate/respiratory rate/blood pressure trend graph; the contents of the 24-hour physiological parameters data sub-page are obtained based on the physiological parameters data and its data labeling;
a 24-hour blood gas analysis and laboratory diagnostic data sub-page, comprising a 24-hour blood gas analysis monitoring record sheet, a partial pressure of oxygen/partial pressure of carbon dioxide/bicarbonate trend graph, and a laboratory diagnostic data sheet;
the contents of the 24-hour blood gas analysis and laboratory diagnostic data sub-page are obtained based on the blood gas analysis data, the laboratory diagnostic data, and their data labeling;
an alarm events data sub-page, comprising: an alarm events record sheet, a 24-hour alarm events distribution diagram, and an alarm events comprehensive analysis sheet;
the alarm events data sub-page is obtained based on the abnormal events labeled by the mechanical ventilation therapy data;
an extended image data sub-page, configured to display the image data.

Further, the report analysis page further comprises a graphic page; the graphic page is divided into a mechanical ventilation waveform data area, a mechanical ventilation loop chart area, and a physiological parameters waveform data area; wherein

the mechanical ventilation waveform data area comprises waveform graphs of continuous normal mechanical ventilation data and waveform graphs of abnormal mechanical ventilation events data;
the mechanical ventilation loop chart area comprises a pressure-volume loop chart and a flow-volume loop chart drawn by the combination of the mechanical ventilation pressure, volume, and flow data;
the contents of the mechanical ventilation waveform data area and the mechanical ventilation loop chart area are obtained based on the mechanical ventilation data and its data labeling;
the physiological parameters waveform data area comprises waveform graphs of continuous physiological parameters data synchronized with the mechanical ventilation data waveform graphs;
the contents of the physiological parameters waveform data area are obtained based on the physiological parameters data and its data labeling.

Further, the report generation instruction is a customized report generation instruction;

the customized report generation instruction comprises one or more customized parameters, configured to generate the report overview page and the report analysis page matching the customized parameters;
the customized parameters comprise the trend graph page, the data page, or the graphic page.

Further, the method further comprises:
in the data labeling process, a first aid instruction is issued when one or more abnormal events of ventilators stop working, asphyxia, increasing airway pressure, cardiac arrest, tachycardia, bradycardia, ventricular flutter, ventricular fibrillation and hypotension occur, the first aid instruction carries the information about the abnormal events that occur.
Further, the method further comprises:
in the data labeling process, a ventilator parameters adjustment instruction is issued when one or more abnormal events of patient-ventilator asynchronous event, high airway pressure, low airway pressure, high positive end expiratory pressure, low positive end expiratory pressure, high minute ventilation, low minute ventilation, high tidal volume, low tidal volume, high respiratory rate, low respiratory rate, high oxygen concentration and low oxygen concentration occur, the ventilator parameters adjustment instruction carries the information about the abnormal events that occur.

Further, in the standard data structure object, a corresponding data structure object is configured for each item of the mechanical ventilation therapy data and the patient information respectively; each decoupled mechanical ventilation therapy data and the patient information are placed into the corresponding data structure object to form standardized data.

Further, while receiving the mechanical ventilation therapy data in real time, streaming media data of the treatment scenes is also received in real time;

live broadcast in real time of the waveform data, the numeric data, and the treatment scenes in the mechanical ventilation therapy data of the medical data set as well as the mechanical ventilation therapy data analysis reports of some or all patients in one or more wards.

In another aspect, the present application also provides a system of mechanical ventilation therapy data management, as defined in claims 11 to 13.

Compared with existing technology, the present application achieves at least one of the following beneficial effects.

The method and system of mechanical ventilation therapy data management provided by the present application has the following advantages.

1. The present application provides technical means for doctors and nurses to centrally manage the mechanical ventilation therapy data, doctors and nurses use clinical data terminal devices to remotely and centrally manage the mechanical ventilation therapy data business of patients in a plurality of wards in a visualized way, providing real-time data monitoring and mechanical ventilation data analysis reports, which improves data work efficiency and mechanical ventilation therapy quality, reduces the labor intensity of doctors and nurses, alleviates the problem of human resources shortage in ICU, solves the defects of the existing mechanical ventilation therapy business, and has wide applicability.

2. The present application decouples the different data communication protocols from various manufacturers' devices from the contained data, separates the multi-source heterogeneous data, parses and filters the huge multi-source heterogeneous data separately, and reconstructs the data into standardized data, which effectively reduces the complexity of the parsing process, improves the parsing speed and stability, solves the defects that the existing communication protocols of the medical devices data and the data formats are confusing and difficult to be uniformly processed, and has wide applicability.

3. The present application aggregates and analyzes the mechanical ventilation therapy data to generate the mechanical ventilation therapy data analysis reports, so that doctors and nurses can conveniently obtain summarized data sets, can quickly and accurately judge the current and past mechanical ventilation therapy status and disease evolution trends, and provide timely intervention measures, solves the difficulties caused by huge multi-source heterogeneous data to the clinical application, reduces the work pressure of doctors and nurses, improves the quality and efficiency of medical treatment, and has wide applicability.

4. The data labeling method provided by the present application can effectively remove the interference data and the data that the device itself analyzes incorrectly, effectively reduce the pressure of the data work of doctors and nurses, and at the same time substantially improve the data quality, solve the defects that the existing devices and clinical information systems store the data of low quality, poor usability, and lack of waveform data, having wide applicability.

In the present invention, the above technical solutions can also be combined, to implement more preferred combined solutions. Other features and advantages of the present invention will be described in the subsequent specification, and part of the advantages can become apparent from the specification, or be understood through the implementation of the present invention. The objects and other advantages of the present invention can be implemented and obtained from the contents particularly illustrated in the specification and the drawings.

## BRIEF DESCRIPTION OF THE DRAWINGS

The drawings are merely for the purpose of illustrating the particular embodiments, and are not considered as limitation to the present application. Throughout the drawings, the same reference signs denote the same elements.

FIG. 1 is a flow chart of the method of mechanical ventilation therapy data management provided in embodiment 1 of the present application.

FIG. 2 is an example diagram of the standard data structure object provided in embodiment 1 of the present application.

FIG. 3 is a schematic diagram of the model input processing during the process of determining the patient-ventilator asynchronous event provided in embodiment 1 of the present application; wherein FIG. 3(a) represents a schematic diagram of the two times sliding window processing;

FIG. 3(b) represents a schematic diagram of the time series after the two times sliding window processing.

FIG. 4 is the report overview page of the mechanical ventilation therapy data analysis provided in embodiment 1 of the present application.

FIG. 5 is the trend graph page of the mechanical ventilation therapy data analysis report provided in embodiment 1 of the present application.

FIG. 6 is the data page of the mechanical ventilation therapy data analysis report provided in embodiment 1 of the present application; wherein, FIG. 6(a) represents a data sub-page of the mechanical ventilation setting parameters; FIG. 6(b) represents a data sub-page of the mechanical ventilation monitoring parameters; FIG. 6(c) represents a data sub-page of the 24-hour physiological parameters; FIG. 6(d) represents data sub-page of the 24-hour blood gas analysis and laboratory diagnostic; FIG. 6(e) represents a data sub-page of the alarm events; FIG. 6(f) represents the image data sub-page.

FIG. 7 is the graphic page of the mechanical ventilation therapy data analysis report provided in embodiment 1 of the present application.

FIG. 8 is an architectural diagram of the system of mechanical ventilation therapy data management with data aggregation of multiple devices provided in embodiment 2 of the present application.

FIG. 9 is an implementation example of the patient displayed in a visualized way provided in embodiment 2 of the present application.

FIG. 10 is an implementation example of the ward management panel of the clinical data terminal devices provided in embodiment 2 of the present application.

FIG. 11 is an implementation example of the data analysis report panel of the clinical data terminal devices provided in embodiment 2 of the present application.

FIG. 12 is an architectural diagram of the system of mechanical ventilation therapy data management in the form of edge computing nodes provided in embodiment 3 of the present application.

## DESCRIPTION OF EMBODIMENTS

The preferable embodiments of the present application will be particularly described below by referring to the drawings. The drawings form part of the present application, are used to explain the principle of the present application together with the embodiments of the present application, and are not limiting the scope of the present application.

Embodiment 1

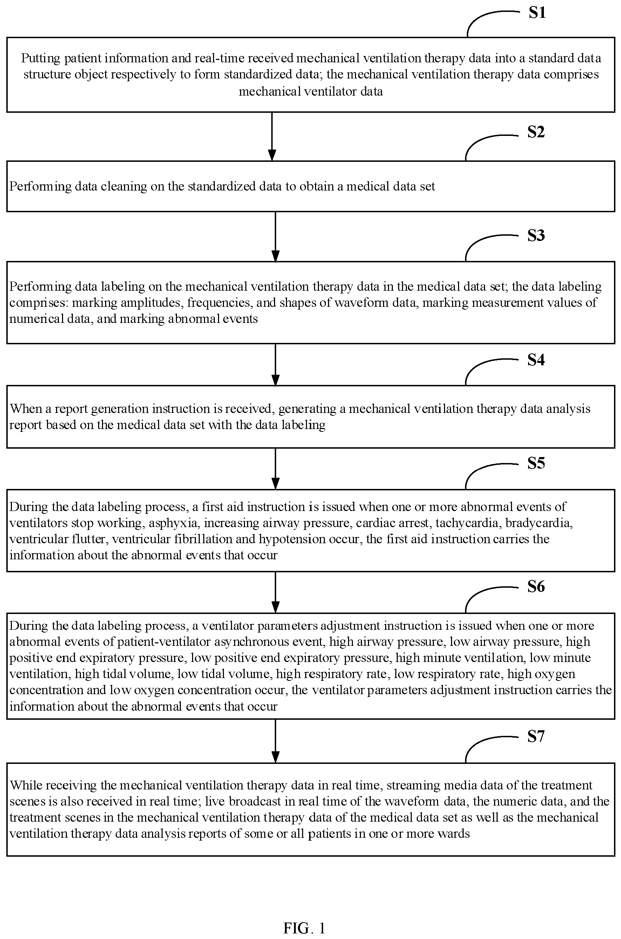

Embodiment 1 of the present application provides a method of mechanical ventilation therapy data management, the flow chart as shown in FIG. 1, comprises the following steps.

Step S1: putting patient information and real-time received mechanical ventilation therapy data into a standard data structure object respectively to form standardized data; the mechanical ventilation therapy data comprises ventilator data.

In step S1, the mechanical ventilation therapy data further comprises one or more of the following data: physiological parameters data, blood gas analysis data, laboratory diagnostic data, imaging data, clinical diagnosis and treatment information, and the ID of the device for outputting the mechanical ventilation therapy data; wherein the physiological parameters data is output by multi-parameter monitors; the patient information, the blood gas analysis data, the laboratory diagnostic data, the imaging data and the clinical diagnosis and treatment information are all output by a clinical information system; the mechanical ventilation data is output by ventilator devices. For example, the clinical information system comprises at least one of HIS, CIS, and EMR systems.

In the specific implementation process, in order to solve the problems that the output data forms of similar devices from different manufacturers as well as of different kinds of mechanical ventilation therapy data acquisition devices are not uniform, resulting in cumbersome subsequent data processing procedure, the present embodiment sets up a standard data structure object so as to perform standardize processing on the multi-source heterogeneous data output from the above devices. Specifically, in the present embodiment, different data communication protocols of the devices from various manufacturers are received and processed in parallel; data messages from the ventilators, the multi-parameter monitors and the clinical information systems are synchronously collected; the head and tail structures and data portions in the data messages are decoupled and separated, wherein the data portions are parsed in parallel, from which the valid data is extracted and filled into the corresponding positions of the preset standard data structure object to form standardized data, which is bundled and encapsulated with the patient information, setting the unique identification, and storing in the database; the standard data structure object is an abstraction of the mechanical ventilation therapy data and the patient information, simplifying the complex multi-source heterogeneous data, thereby improving the efficiency of the data analysis, processing, and utilization, reducing the cost of data application.

Therefore, the present embodiment can complete the standardization process of the multi-source heterogeneous data by performing data parsing and standardization processing, reduce the complexity of parsing multi-source heterogeneous data, improve the speed and stability of data parsing, the standardized data can not only conserve storage space, but also improve the efficiency of data computing and retrieval.

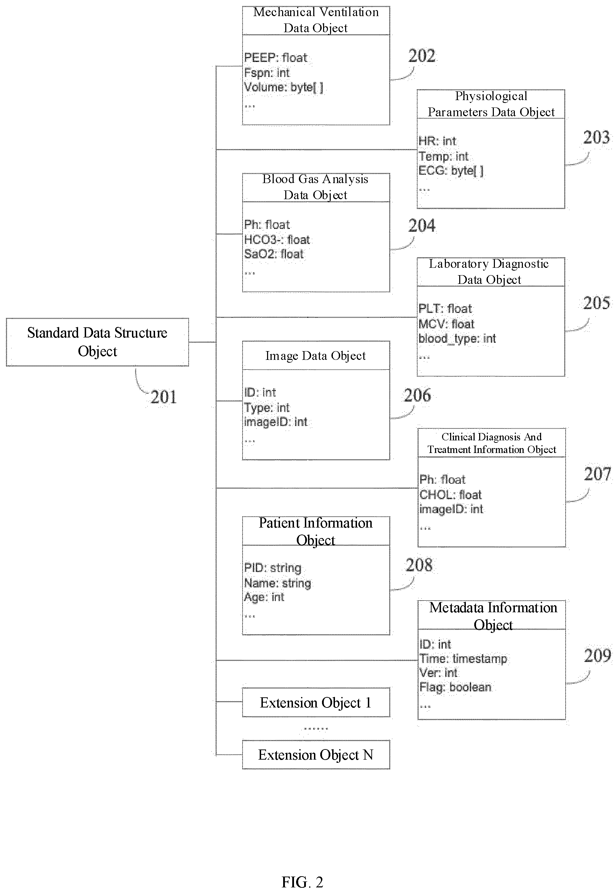

FIG. 2 illustrates an example of a standard data structure object 201, comprising a mechanical ventilation data object 202, a physiological parameters data object 203, a blood gas analysis data object 204, a laboratory diagnostic data object 205, an image data object 206, a clinical diagnosis and treatment information object 207 and a patient information object 208, the specific contents of each data structure object can be preset according to the actual situation. In addition, FIG. 2 further comprises a metadata information object 209, configured to describe the standard data structure object 201 itself and the contained structures, contents and attributes of the data structure object, and define the names, relationships, fields, constraints and syntactic structures of each data structure object. Since the blood gas analysis data, the laboratory diagnostic data, the image data, etc. are all periodic data that occur intermittently or at regular intervals during the mechanical ventilation therapy process, the standard data structure object 201 allows to be set to null when the relevant data does not occur. In addition, in order to enhance the adaptability and extensibility of the standard data structure object 201, the standard data structure object 201 comprises the extension objects 1-n as shown in FIG. 2, which are configured to add other relevant mechanical ventilation therapy data. For example, for a shock patient with mechanical ventilation therapy, filtration blood of CRRT and dynamic monitoring of urine volume need to be performed at the same time, at this time, the mechanical ventilation therapy data can also comprise continuous blood filtration data and dynamic monitoring data of urine volume, accordingly, the standard data structure object 201 can extend the continuous blood filtration data object and the dynamic monitoring data object of urine volume, so as to add other relevant mechanical ventilation therapy data conveniently. The standard data structure object provided by the present embodiment effectively solved the defects that the existing communication protocols of the medical devices data and the data formats are confusing and difficult to be uniformly processed, and have wide applicability. Specifically, the standard data structure object can be implemented by high-level languages, taking the object-oriented programming language JAVA as an example, the standard data structure object is a specific class, the attributes (fields) of the class are the various data structure objects, the various method functions of the class are programmed for external calls, configured to set the specific data structure objects and their contained parameters, for example, airway resistance and work of breathing represented by *Float* type, volume waveform data represented by *LinkedList* type, attribute identifiers represented by Boolean type. The standard data structure object class is distributed to the parsing programs, each parsing program creates a standard data structure class object for each data packet when parsing in parallel, calls the methods of the class to fill the data, and combines the standard data structure class objects at the same time into a single standardized data through the aggregation program.

Step S2: performing data cleaning on the standardized data to obtain a medical data set.

Data cleansing is a process for re-examining and verification data, aiming to delete duplicate information, correct existing errors, and provide data consistency. The existing data cleaning techniques are relatively mature, in the actual implementation process, a corresponding data cleaning method can be selected according to the specific application scene and the specific situation of the mechanical ventilation therapy data under the application scene. For example, the data cleaning process in the present embodiment can comprise the following operations:

(1) judgment of data constraint range: range constraints on medical theory data values, data constraints on empiricist extrapolations;
(2) conversion of data unit/data precision;
(3) removal of data outlier;
(4) for waveform data, also performing data length calibration, data integrity verification, etc.; the medical data set obtained after data cleaning can be used for subsequent data analysis.

Step S3: performing data labeling on waveform data and numerical data of the mechanical ventilation therapy data; the data labeling comprises: marking amplitudes, frequencies, and shapes of waveform data, marking measurement values of numerical data, and marking abnormal events; wherein the measurement values of the labeled numerical data can comprise parameters that are higher than the upper limit threshold of the corresponding numerical data or lower than the lower limit threshold of the corresponding numerical data; the labeled data is automatically stored into the medical data set.

Since the mechanical ventilation therapy data can contain many specific data that cannot be exhaustively listed, in the present embodiment, only common data and common data labeling in various mechanical ventilation therapy data are highlighted.

Specifically, (1) in the mechanical ventilation data, the waveform data comprises: mechanical ventilation pressure waveform data, volume waveform data, flow waveform data and end-tidal carbon dioxide waveform data; the numerical data comprises: airway resistance, work of breathing, compliance, ventilation parameters and respiratory rate; in the data labeling process for the mechanical ventilation data, the shapes of the labeled waveform data comprises: beginning of inspiratory phase, inspiratory phase, end of inspiratory phase, beginning of expiratory phase, expiratory phase, end of expiratory phase, peak airway pressure, and peak expiratory flow rate of each mechanical ventilation waveform, as well as inspiratory baseline, expiratory plateau height, and expiratory plateau beginning and end of the end-tidal carbon dioxide waveform; the labeled contents can be configured to generate the report overview page and the report analysis page either directly or after statistics, computation and analysis; for example, the peak airway pressure and the peak expiratory flow rate can be determined by the following process.

(a) Firstly, common filtering algorithms such as median/homogenous filtering are adopted to filter and aggregate the mechanical ventilation pressure, volume and flow waveform data having high sampling rate in order to reduce the complexity of waveform processing. A pressure-time curve of the mechanical ventilation pressure waveform data is recorded as $P(t)$, a volume-time curve of the mechanical ventilation volume waveform data is recorded as $V(t)$, a flow-time curve of the mechanical ventilation flow waveform data is recorded as $F(t)$; setting the observation window, for example, it takes at least 3.428s for two breaths, so the observation window duration is longer than 3.428s, for example, the observation window can be set to 4s. The following steps (b)-(d) are all calculated according to the observation window.

(b) In the i-th observation window, performing the following process to the volume-time curve $V(t)$:
finding the peaks and valleys within the observation window using the peak finding algorithm and storing the results, and obtaining:

the i-th inspiratory start time $t_i^{ins}$, the i-th inspiratory end time $t_i^{exp}$, wherein $t_i^{exp}$ is also the i-th expiratory start time;

the i-th expiratory end time $t_{i+1}^{ins}$, wherein $t_{i+1}^{ins}$ is also the i+1-th inspiratory start time.

(c) According to the above start and end time of the inspiratory phase and the expiratory phase, performing the calculation as follows to the pressure-time curve $P(t)$ to obtain the peak airway pressure PIP, and storing the results; the i-th peak airway pressure:

$$PIP_i = Max(P(t) | t \in (t_i^{ins}, t_i^{exp}))$$ ; t represents the time.

(d) According to the above start and end time of inspiratory phase and expiratory phase, performing the calculation as follows to the flow-time curve $F(t)$ to obtain the peak expiratory flow rate PEF, and storing the results; the i-th peak expiratory flow rate is:

$$PEF_i = Min(F(t) | t \in (t_i^{exp}, t_{i+1}^{ins}))$$ .

The measurement values of the labeled numerical data comprise: parameters that are higher than the upper limit threshold of the corresponding numerical data or lower than the lower limit threshold of the corresponding numerical data; the labeled content can be configured to generate the trend graph page, sub-page of the mechanical ventilation setting parameters and sub-page of the mechanical ventilation monitoring parameters either directly or after statistics, computation and analysis; for example, numerical comparison is continuously performed between the compliance data and its corresponding upper and lower limit thresholds, when the compliance data is below the lower limit threshold (e.g., the lower limit threshold is 25ml/cmH$_2$O), the compliance data and the occurrence time at that moment are labeled and stored.

The labeled abnormal events comprise: patient-ventilator asynchronous event, high/low airway pressure, high/low positive end-expiratory pressure, high/low expired tidal volume, high/low minute ventilation, high/low inspired oxygen concentration, high/low oxygen concentration, high/low respiratory rate, high/low partial pressure of end-tidal carbon dioxide, high/low tidal volume, ventilators stop working, asphyxia and increasing airway pressure. The labeled contents can be configured to generate the report overview page, the alarm events data sub-page, and the graphic page either directly or after statistics, computation and analysis.

For example, the patient-ventilator asynchronous event can be determined based on a neural network model, the specific process is described as below.

(a) Constructing the model inputs based on the pressure-time curve $P(t)$, the volume-time curve $V(t)$, and the flow-time curve $F(t)$.

Specifically, the pressure-time curve $P(t)$, the volume-time curve $V(t)$, and the flow-time curve $F(t)$ form a three-dimensional time series as the model inputs: $T = \langle S_1, \cdots S_c, \cdots, S_C \rangle$, wherein $S_c$ represents the input vector of the c-th sampling point, $C$ represents the total number of the sampling points processed by the neural network model in a single run, that is, the length of the model inputs; $S_c = \left( S_c^1, S_c^2, S_c^3 \right)$, $S_c^1$, $S_c^2$, $S_c^3$ represents the values of flow rate, pressure, and volume at the c-th sampling point respectively.

(b) Performing two times sliding window processing for feature enrichment to obtain the time series after sliding window processing.

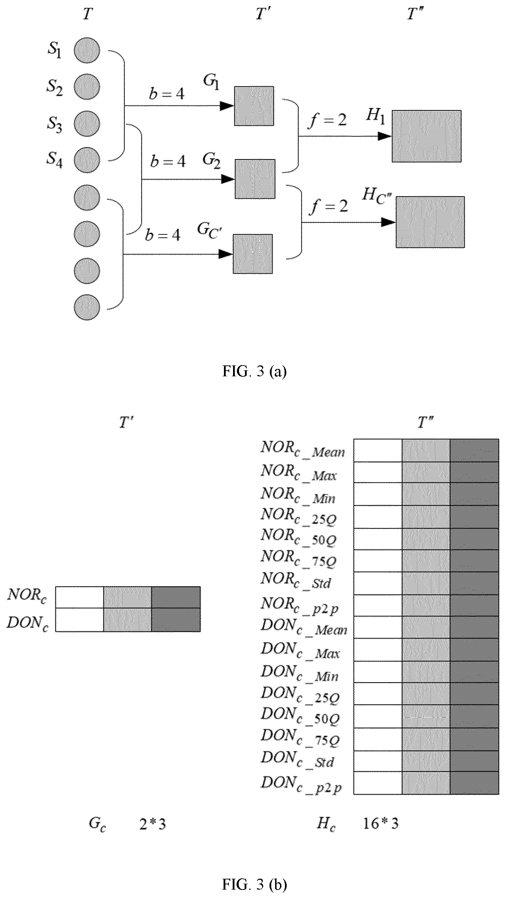

Two times sliding window processing: considering the intrinsic dependence of the time series data, the present embodiment adopts two times sliding window to process the sequence data in pieces, and extract the statistical features of each window, to exclude interfering factors and enhance the feature rules of the time series, thereby better describing the centralized tendency and discrete degree of the distribution of the data values of the waveform sampling points, the skewness and kurtosis features of the waveform shape changes, so that it is convenient to capture the dependency features and local features of the input sequence subsequently. The schematic diagram of the two times sliding window processing is shown in FIG. 3(a).

Wherein, the first sliding window processing, executing the following steps:

performing the sliding window processing to the model input $T$ with b/2 as the sliding window step, $b$ represents the width of the first sliding window; after the first sliding window processing,

the model input length is changed from $C$ to $C'$, the values range of c is changed to [1, C']; $C' = \dfrac{2*C - b}{b}$;

after the first sliding window processing, based on the class $l$ waveform data in all sampling points within the c-th sliding window, the Euclidean distance $NOR_c^l$ of the class $l$ waveform data in the Euclidean matrix vector is calculated, that is, $NOR_c^l = \sqrt{ \left( S_c^l \right)^2 + \left( S_{c+1}^l \right)^2 + \cdots + \left( S_{c+b-1}^l \right)^2 }$, $l$ takes 1, 2, 3 respectively;

finally, the Euclidean matrix vector $NOR_c = \left( NOR_c^1, NOR_c^2, NOR_c^3 \right)$ is obtained.

Based on the class $l$ waveform data in all sampling points within the c-th sliding window, the distance difference $DON_c^l$ of the class $l$ waveform data in the distance difference vector is calculated, that is,

$$DON_c^l = NOR_{c+1}^l - NOR_c^l ;$$

finally, the distance difference vector is obtained and represented as $DON_c = \left( DON_c^1, DON_c^2, DON_c^3 \right)$;

therefore, the time series after the first sliding window processing:
$T' = \langle G_1, \cdots, G_c, \cdots, G_C \rangle$, wherein, $G_c = NOR_c, DON_c \rangle$.

The second sliding window processing, executing the following steps:

performing the sliding window processing to the time series $T'$ again with $f/2$ as the sliding window step, $f$ represents the width of the second time sliding window; after the second time sliding window processing, the model input length is changed from $C'$ to $C''$, the values range of c is changed to [1, C''];

$$C'' = \dfrac{2*C' - f}{f} ;$$

during the second sliding window processing, the derived features of all the Euclidean matrix vectors and distance difference vectors within the second time sliding window can be obtained,
which can be illustrated by taking the c-th sliding window as an example, we can obtain:

the mean vector of the Euclidean matrix and the distance difference ($NOR_{c\_Mean}$, $DON_{c\_Mean}$),

the maximum vector of the Euclidean matrix and the distance difference ($NOR_{c\_Max}$, $DON_{c\_Max}$),
the minimum vector of the Euclidean matrix and the distance difference ($NOR_{c\_Min}$, $DON_{c\_Min}$),
the upper quartile vector of the Euclidean matrix and the distance difference ($NOR_{c\_25Q}$, $DON_{c\_25Q}$),
the median vector of the Euclidean matrix and the distance difference ($NOR_{c\_50Q}$, $DON_{c\_50Q}$),
the lower quartile vector of the Euclidean matrix and the distance difference ($NOR_{c\_75Q}$, $DON_{c\_75Q}$),
the standard deviation vector of the Euclidean matrix and the distance difference ($NOR_{c\_Std}$, $DON_{c\_Std}$)
the deviation vector of the Euclidean matrix and the distance difference ($NOR_{c\_p2p}$, $DON_{c\_p2p}$),

the $l$-th result $NOR_{c\_Mean}^l$ in ($NOR_{c\_Mean}$ is obtained by averaging the Euclidean distances $NOR_c^l$, $NOR_{c+1}^l, \cdots, NOR_{c+f-1}^l$ of the class $l$ waveform data in the Euclidean matrix vector within the c-th sliding window, therefore,

$$NOR_{c\_Mean} = \left( NOR_{c\_Mean}^1, NOR_{c\_Mean}^2, NOR_{c\_Mean}^3 \right);$$

the $l$-th result $DON_{c\_Mean}^l$ in $DON_{c\_Mean}$ is obtained by averaging the distance differences $DON_c^l$, $DON_{c+1}^l, \cdots, DON_{c+f-1}^l$ of the class $l$ waveform data in the distance difference vector within the c-th sliding window, therefore,

$$DON_{c\_Mean} = \left( DON_{c\_Mean}^1, DON_{c\_Mean}^2, DON_{c\_Mean}^3 \right);$$

other derived features after the second time sliding window processing can be determined based on their feature names, the corresponding computation process is performed on $NOR_c^l$, $NOR_{c+1}^l, \cdots, NOR_{c+f-1}^l$, and will not be described again here.

Therefore, the time series after the second time sliding window processing $T'' = \langle H_1, \cdots, H_c, \cdots, H_{C''} \rangle$, wherein

$$H_c = \left\langle \begin{array}{l} NOR_{c\_Mean}, NOR_{c\_Max}, NOR_{c\_Min}, NOR_{c\_25Q}, \\ NOR_{c\_50Q}, NOR_{c\_75Q}, NOR_{c\_Std}, NOR_{c\_p2p}, \\ DON_{c\_Mean}, DON_{c\_Max}, DON_{c\_Min}, DON_{c\_25Q}, \\ DON_{c\_50Q}, DON_{c\_75Q}, DON_{c\_Std}, DON_{c\_p2p} \end{array} \right\rangle.$$

A schematic diagram of the time series after the two times sliding window processing is shown in FIG. 3(b).

(c) The time series after the sliding window processing are input into the neural network model, after processing, the neural network model outputs normal events as well as ventilator abnormal events (specifically, the types of patient-ventilator asynchronous events and their occurrence times); the types of ventilator abnormal events comprise ineffective effort, double triggering, auto-triggering, reverse triggering, flow starvation, overshooting, delayed cycling and premature cycling;

based on the output category results of the neural network model, the total number of patient-ventilator asynchronous and the index of patient-ventilator asynchronous can also be calculated, which can be calculated according to the existing methods and will not be described again here. (2) In the physiological parameters data, the waveform data comprises: electrocardiogram waveform data, respiratory waveform data, photoplethysmogram waveform data and invasive arterial pressure waveform data; the numerical data comprises: heart rate, blood pressure, blood oxygen saturation and body temperature; during the data labeling process for the physiological parameters data.

The shapes of the labeled electrocardiogram waveform data comprise: P wave, QRS complex, T wave, ST segment, PR interval, RR interval and QT interval of each ECG cycle waveform; the shapes of the labeled respiratory waveform data comprise: peaks and valleys of each respiratory cycle waveform; the shapes of the labeled photoplethysmogram waveform data comprises: peaks and valleys of each pulse cycle waveform; the shapes of the labeled invasive arterial pressure waveform data comprises: peaks and valleys of each arterial systolic-diastolic cycle waveform; the labeled contents can be configured to generate the report overview page and the report analysis page either directly or after statistics, computation and analysis.

The measurement values of the labeled numerical data comprise: parameters that are higher than the upper limit threshold of the corresponding numerical data or lower than the lower limit threshold of the corresponding numerical data; the labeled contents can be configured to generate the 24-hour physiological parameters data sub-page either directly or after statistics, computation and analysis.

The labeled abnormal events comprise: tachycardia, bradycardia, arrhythmia, cardiac arrest, atrial flutter, atrial fibrillation, ventricular flutter, ventricular fibrillation, atrioventricular block, ST-segment elevation/depression, high/low heart rate, high/low blood pressure, high/low body temperature and low blood oxygen saturation; the labeled contents can be configured to generate the report overview page, the report analysis page and the alarm events data sub-page either directly or after statistics, computation and analysis.

(3) In the blood gas analysis data, the numerical data comprises: partial pressure of oxygen, partial pressure of carbon dioxide, total carbon dioxide, potassium ion concentration, sodium ion concentration, bicarbonate ion concentration and pH value.

In the data labeling process for the blood gas analysis data,
the measurement values of the labeled numerical data comprise: parameters that are higher than the upper limit threshold of the corresponding numerical data or lower than the lower limit threshold of the corresponding numerical data; the labeled contents can be configured to generate the 24-hour blood gas analysis and laboratory diagnostic data sub-page either directly or after statistics, computation and analysis.

The labeled abnormal events comprise: high/low partial pressure of oxygen, high/low partial pressure of carbon dioxide, high/low total carbon dioxide, high/low potassium ion concentration, high/low sodium ion concentration, high/low bicarbonate ion concentration and high/low pH value. The labeled contents can be configured to generate the 24-hour blood gas analysis and laboratory diagnostic data sub-page either directly or after statistics, computation and analysis. (4) In the laboratory diagnostic data, the numerical data comprises: white blood cell count, red blood cell count, platelet count, hemoglobin, hematocrit, blood glucose, prothrombin time and activated partial thrombin time.

In the data labeling process for the laboratory diagnostic data,
the measurement values of the labeled numerical data comprise: parameters that are higher than the upper limit threshold of the corresponding numerical data or lower than the lower limit threshold of the corresponding numerical data; the labeled contents can be configured to generate the 24-hour blood gas analysis and laboratory diagnostic data sub-page either directly or after statistics, computation and analysis.

The labeled abnormal events comprise: high/low white blood cell count, high/low red blood cell count, high/low hemoglobin, high/low hematocrit, high/low blood glucose, high/low platelet count, long/short prothrombin time, and long/short activated partial thrombin time. The labeled contents can be configured to generate the 24-hour blood gas analysis and laboratory diagnostic data sub-page either directly or after statistics, computation and analysis.

Other unexhausted mechanical ventilation therapy data and their data labeling are also easily conceived by a person skilled in the art based on the solution of the present embodiment, and therefore, also fall within the scope of protection of the present application.

Step S4: when a report generation instruction is received, generating a mechanical ventilation therapy data analysis report based on the medical data set with the data labeling.

For example, in step S4, retrieving the medical data set with the data labeling, performing the multi-dimensional data aggregation analysis to generate the analysis conclusion based on correlation, while various types of data are combined with the time labels and dimension labels and filled into the preset data analysis report framework to generate the mechanical ventilation therapy data analysis report, which is stored in the database. In addition, in order to further ensure the accuracy of the mechanical ventilation therapy data analysis report, manual review operation can be added, the mechanical ventilation therapy data analysis report after manual review is stored in the database.

It should be noted that the various types of data in the medical data set are computed, analyzed, and counted layer by layer to obtain data that meets the required contents and accuracy requirements in the preset framework of the analysis report, configured to generate the mechanical ventilation therapy data analysis report.

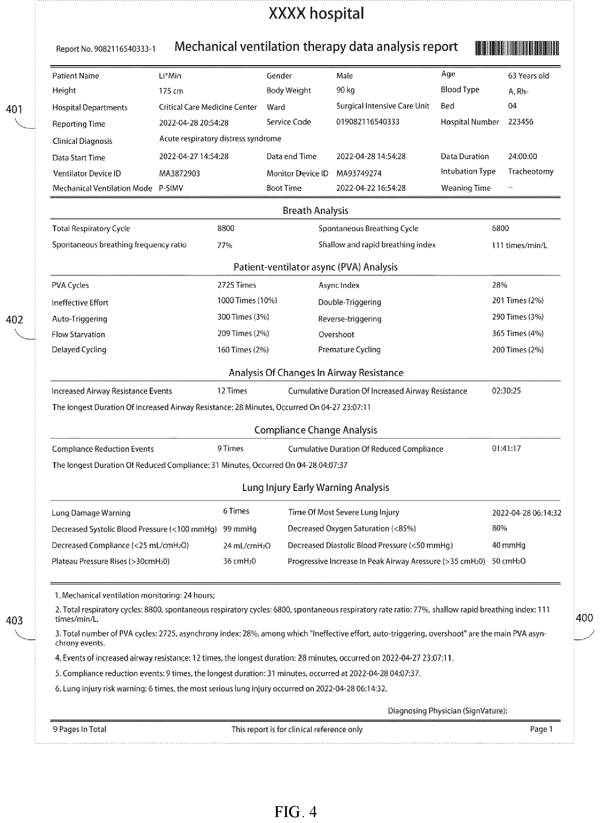

The reporting framework of the mechanical ventilation therapy data analysis report comprises a report overview page and a report analysis page; wherein as shown in FIG. 4, the report overview page 400 is divided into a basic information area 401, a mechanical ventilation data area 402, and an analysis conclusion area 403.

The basic information area 401 comprises at least patient information and key time point information; and further comprises the following options: the clinical diagnosis information, the ID of the device for outputting the mechanical ventilation therapy data as well as mechanical ventilation mode and setting information; for example, the patient information can comprise hospital information, basic patient information, ward beds information, hospital departments, etc.; the key time point information can comprise report time, data start time, data end time, device start up time, device shut down time, etc..

The mechanical ventilation data area 402 comprises at least respiratory analysis results, patient-ventilator asynchronous events analysis results, airway resistance change analysis results, compliance change analysis results, and lung injury early warning analysis results; each of the analysis results is obtained based on the mechanical ventilation data and its data labeling; for example, the respiratory analysis results comprise: a total respiratory cycle, a spontaneous breathing cycle, a proportion of spontaneous breathing frequency and a rapid shallow breathing index.

Specifically, in the present embodiment, the total respiratory cycles, spontaneous breathing cycles and tidal volume labeled for the mechanical ventilation waveform data are statistically computed to obtain the number of total respiratory cycles, the number of spontaneous breathing cycles, the proportion of spontaneous breathing frequency, and the rapid shallow breathing index, configured to evaluate the possibility of the patient going offline. The data labeled for the mechanical ventilation patient-ventilator asynchronous events is statistical computed to obtain the total number of the patient-ventilator asynchronous events, the frequency of each type of asynchronous events is counted and its proportion is calculated. The compliance data labeled for the mechanical ventilation numerical data is computed to obtain the value and occurrence time of compliance reduction, and counts the number of times of compliance reduction, cumulative duration and the longest reduction duration within the preset time range. FIG. 4 shows an example of each analysis result in the mechanical ventilation data area, these example contents are the indicator data that clinicians are more concerned about and have high clinical value.

The analysis conclusion area 403 in FIG. 4 is configured to display the analysis descriptions and conclusions of the mechanical ventilation therapy data; wherein the analysis conclusion area can comprise the conclusion of the mechanical ventilation monitoring time measurement, the analysis conclusion of the respiratory (comprising the "rapid shallow breathing index"), the analysis conclusion of the patient-ventilator asynchronous events, the analysis conclusion of the airway resistance changes, the analysis conclusion of the compliance change, and the analysis conclusion of the lung injury early warning. Specifically, the various types of data in the mechanical ventilation data area are counted to obtain the two major categories of data such as normal interval and abnormal interval; the events are stratified according to the number of occurrence, the time of occurrence, the duration of the events in the abnormal interval, the type and the risk degree of the events in the abnormal interval, and are associated with the corresponding various types of analysis conclusion items to describe the state of the patient's mechanical ventilation therapy, generate analysis conclusions, and fill into the preset data analysis report framework. For example, the analysis conclusion of compliance change counts the number of occurrences of the events that meet the preset compliance reduction abnormal interval (<25 ml/cmH$_2$O) according to the labeled compliance reduction event data, and compares the duration of all the compliance change abnormal events to obtain the longest lasting compliance reduction event. Finally, the analytic conclusions were generated according to the number of occurrences of compliance reduction events, the longest lasting compliance reduction event and the time of occurrence, then filled into the analytic conclusion area 403.

The report analysis page is configured to display a detailed analysis report of one or more mechanical ventilation therapy data separately in graphical form.

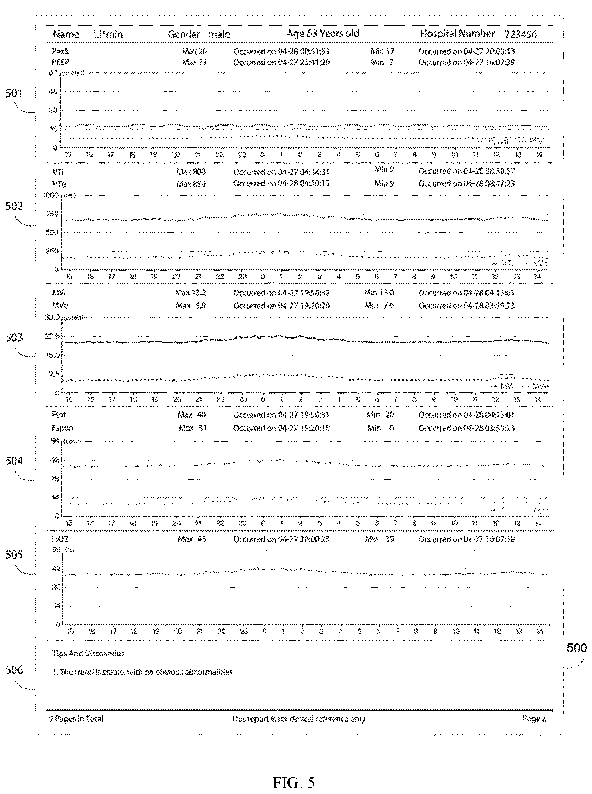

The report analysis page comprises a trend graph page 500, as shown in FIG. 5; the trend graph page comprises: a pressure trend analysis area 501, a volume trend analysis area 502, a ventilation trend analysis area 503, a trend analysis area of a respiratory frequency 504, a trend analysis area of an inhaled oxygen concentration 505, and a trend analysis description and conclusion area 506 of the mechanical ventilation data. Wherein the trend analysis description and conclusion area 506, configured to synthesize the trend direction of each trend analysis area to provide trend analysis descriptions and the corresponding conclusions. In addition, the contents of each trend analysis area are obtained based on the mechanical ventilation data and its data labeling, dividing into 24 number of hour data units by hour, calculating the average value of the parameters related with the report in each unit, and drawing the trend graph by plotting the average value of hourly parameters with a line.

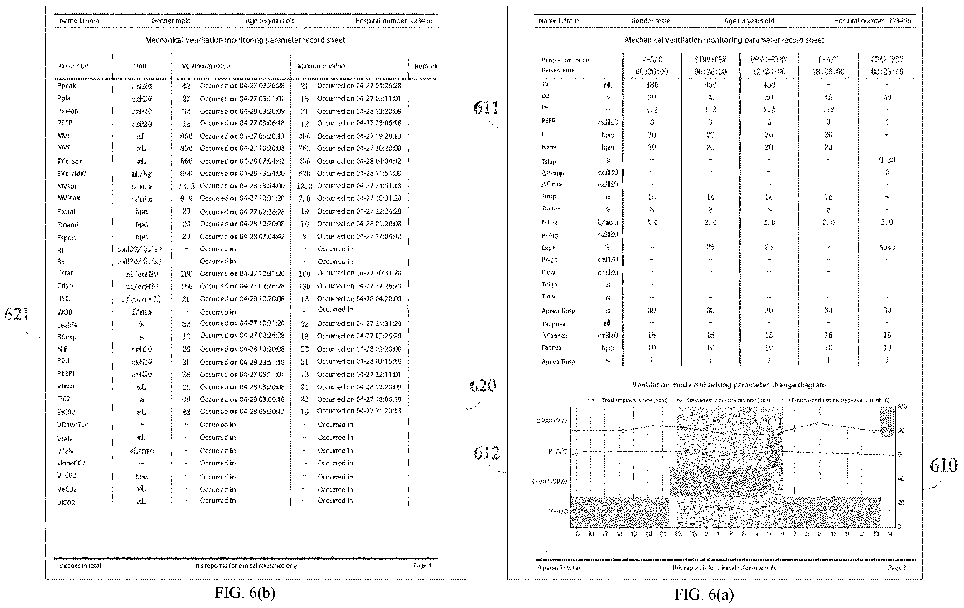

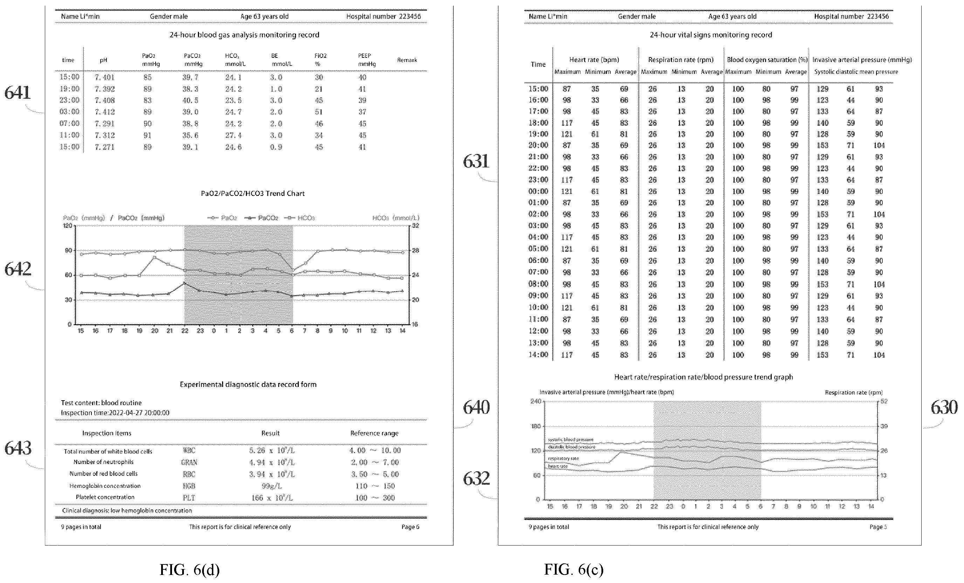

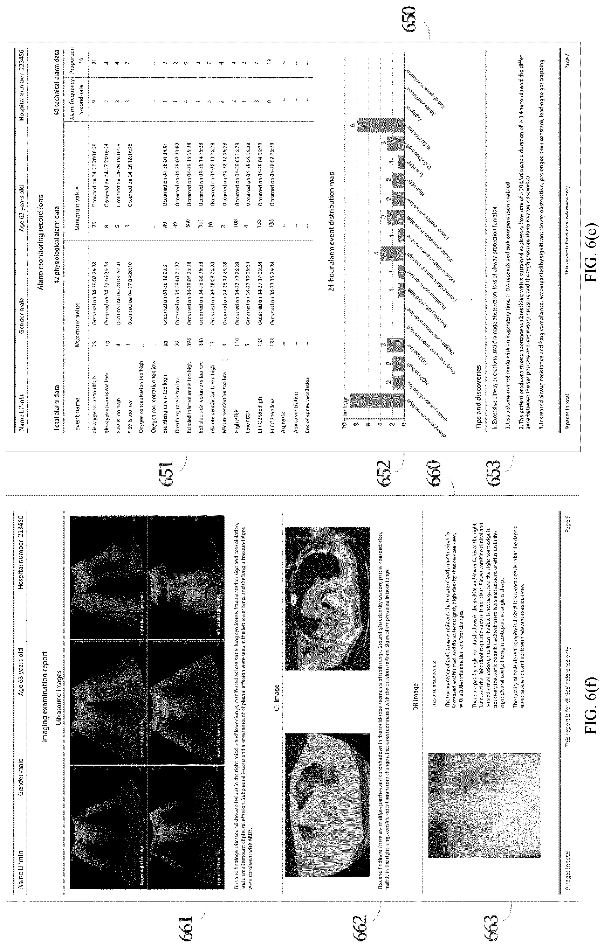

The report analysis page further comprises a data page, as shown in FIG. 6; the data page comprises at least: a mechanical ventilation setting parameters data sub-page 610, a mechanical ventilation monitoring parameters data sub-page 620; the data page further comprises the following optional data sub-pages: a 24-hour physiological parameters data sub-page 630, a 24-hour blood gas analysis and laboratory diagnostic data sub-page 640, and an alarm events data sub-page 650.

Wherein, the mechanical ventilation setting parameters data sub-page 610, as shown in FIG. 6(a), comprising a mechanical ventilation setting parameters record sheet 611, ventilation modes of mechanical ventilation and a change trend graph of setting parameters 612.

The mechanical ventilation monitoring parameters data sub-page 620, as shown in FIG. 6(b); comprising a mechanical ventilation monitoring parameters record sheet 621; the contents of the mechanical ventilation setting parameters data sub-page and the mechanical ventilation monitoring parameters data sub-page 620 are obtained based on the mechanical ventilation data and its data labeling.

The 24-hour physiological parameters data sub-page 630, as shown in FIG. 6(c); comprising a 24-hour vital signs monitoring record sheet 631 and a heart rate/respiratory rate/blood pressure trend graph 632; the contents of the 24-hour physiological parameters data sub-page 630 are obtained based on the physiological parameters data and its data labeling.

The 24-hour blood gas analysis and laboratory diagnostic data sub-page 640, as shown in FIG. 6(d); comprising a 24-hour blood gas analysis monitoring record sheet 641, a partial pressure of oxygen/partial pressure of carbon dioxide/bicarbonate trend graph 642, and an laboratory diagnostic data sheet 643; the contents of the 24-hour blood gas analysis and laboratory diagnostic data sub-page 640 are obtained based on the blood gas analysis data, the laboratory diagnostic data, and their data labeling.

The alarm events data sub-page 650, as shown in FIG. 6(e); comprising an alarm events record sheet 651, a 24-hour alarm events distribution diagram 652, and an alarm events comprehensive analysis sheet 653. The contents of the alarm events data sub-page 650 are obtained based on the abnormal events labeled by the mechanical ventilation therapy data.

The extended image data sub-page 660, as shown in FIG. 6(e); configured to display the image data. For example, the image data can comprise an ultrasound image examination area 661, a CT image examination area 662, and a DR image examination area 663.

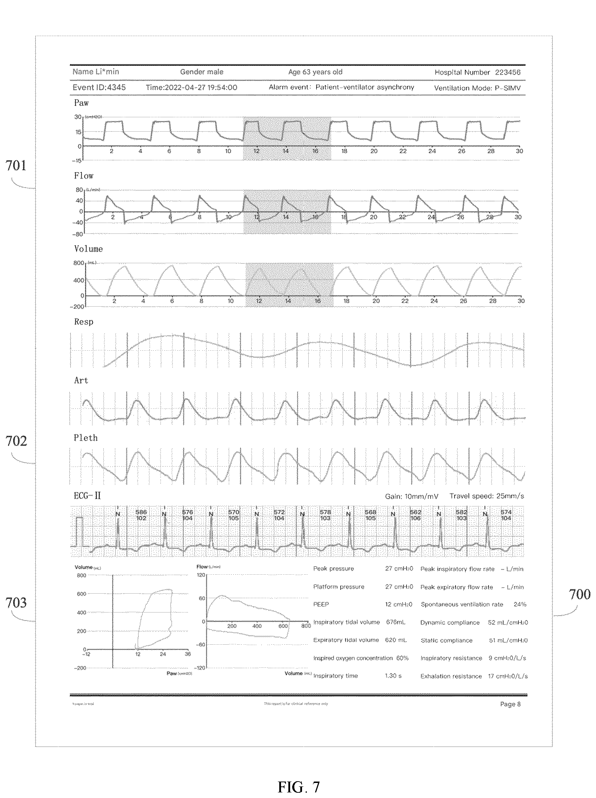

The report analysis page further comprises a graphic page 700, as shown in FIG. 7; the graphic page is divided into a mechanical ventilation waveform data area 701, a physiological parameters waveform data area 702, and a mechanical ventilation loop chart area 703.

Wherein, the mechanical ventilation waveform data area 701 comprises waveform graphs of continuous normal mechanical ventilation data and waveform graphs of abnormal mechanical ventilation events data, and the waveform graphs have waveform measurement values, labeling of each waveform cycle and labeled types of the abnormal events attached to them.

The physiological parameter waveform data area 702 comprises waveform graphs of continuous physiological parameters data synchronized with the mechanical ventilation data waveform graphs, and the waveform graphs have waveform measurement values, labeling of each waveform cycle and labeled types of the abnormal events. The contents of the physiological parameter waveform data area are obtained based on the physiological parameters data and its data labeling.

The mechanical ventilation loop chart area 703 comprises a pressure-volume loop chart and a flow-volume loop chart drawn by the combination of the pressure, volume, and flow data within a mechanical ventilation event cycle, and highlighting the abnormal loop chart in a superimposed manner; the contents of the mechanical ventilation waveform data area and the mechanical ventilation loop chart area are obtained based on the mechanical ventilation data and its data labeling; for example, using the mechanical ventilation data to plot the normal mechanical ventilation data waveform graphs, the abnormal mechanical ventilation event data waveform graphs, and the mechanical ventilation loop charts, and superimposing the loop charts formed by each mechanical ventilation, highlighting the loop charts with changes in morphology, and labeling the time of occurrence and duration.

The above graphic page can be laid out according to the order of the mechanical ventilation waveform data area and the physiological parameters waveform data area; the order of the mechanical ventilation waveform data and the physiological parameters waveform data can also be reorganized according to the data usage habits and thinking ways of clinician.

It should be emphasized that the mechanical ventilation therapy data analysis report is a multi-types, multi time series summarized data set, which provides summarized analyses and conclusions of the key indexes data of the mechanical ventilation therapy, as well as various key indexes data, abnormal events data, and evolutionary trends, comprising physiological parameters data, blood gas analysis data and laboratory diagnostic data during the therapy; and provides waveform graphic data that reflects the state of ventilator's function, the state of the patient/ventilator interaction, the state of respiratory mechanics, the state of physiological parameters, the waveform graphic data of all kinds of event state; can also provide the image data of lung, heart, and abdominal diaphragm. Based on the mechanical ventilation therapy data analysis report, doctors and nurses can quickly grasp the comprehensive mechanical ventilation therapy data, disease status and evolution trend, evaluate the effectiveness of the treatment strategy, provide timely intervention measures to reduce lung injury and other complications, improved the efficiency of data processing and the quality of mechanical ventilation therapy, promoted the recovery of patients, which has wide applicability.

In actual application, in order to extend the application scenes of the solution in the present embodiment, the report generation instruction can be a customized report generation instruction; the customized report generation instruction comprises one or more customized parameters, configured to generate the report overview page and the report analysis page matching the customized parameters; the customized parameters comprise the trend graph page, the data page, or the graph page. For example, when only the trend graph page is comprised in the customized parameters, the report analysis page in the generated mechanical ventilation therapy data analysis report also comprises only the trend graph page and no longer comprises the data page and the graphic page. The generation of each item in the mechanical ventilation therapy data analysis report can be referred to the above description and will not be described again here.

In addition, the method further comprises:

Step S5: in the data labeling process, a first aid instruction is issued when one or more abnormal events of ventilators stop working, asphyxia, increasing airway pressure, cardiac arrest, tachycardia, bradycardia, ventricular flutter, ventricular fibrillation and hypotension (shock) occur, the first aid instruction carries the information about the abnormal events that occur.

Step S6: the method further comprises:

in the data labeling process, a ventilator parameters adjustment instruction is issued when one or more abnormal events of patient-ventilator asynchronous event, high airway pressure, low airway pressure, high positive end expiratory pressure, low positive end expiratory pressure, high minute ventilation, low minute ventilation, high tidal volume, low tidal volume, high respiratory rate, low respiratory rate, high oxygen concentration and low oxygen concentration occur, the ventilator parameters adjustment instruction carries the information about the abnormal events that occur.

In addition, step S7: the method further comprises:

while receiving the mechanical ventilation therapy data in real time, streaming media data of the treatment scenes is also received in real time (for example, the streaming media data can be collected by bedside audio video devices); is live broadcast in real time of the waveform data, the numeric data, and the treatment scenes in the mechanical ventilation therapy data of the medical data set, as well as the mechanical ventilation therapy data analysis reports of some or all patients in one or more wards. For example, the clinical data terminal devices can live broadcast the mechanical ventilation waveform data, the numeric data, and the streaming media data of the medical data set. When the data is derived from a plurality of patients in a plurality of wards, the data and treatment scenes of patients in the plurality of wards are displayed in a visualized way; furthermore, the streaming media data can be associated with the medical data set, all of which are stored into the database.

The present embodiment provides a method of mechanical ventilation therapy data management, doctors and nurses remotely and centrally can manage the mechanical ventilation therapy data business of a plurality of wards in a visualized way, process a huge amount of heterogeneous data generated by a plurality of devices into data with standardized data structure to generate the mechanical ventilation therapy data analysis reports. Doctors and nurses are able to observe and analyze the real-time data, events data, data analysis reports, treatment scenes of the mechanical ventilation therapy of patients in a plurality of wards, comprising the laboratory diagnostic data and the image data from the clinical information systems, on a single clinical data terminal device, master the current and past mechanical ventilation therapy status and disease evolution trend, discover complications at an early stage, and provide timely intervention measures to improve the quality of mechanical ventilation therapy and work efficiency, reduce the labor intensity of doctors and nurses, solve the difficulties caused by huge multi-source heterogeneous data to the clinical application, and has wide applicability.

**Embodiment 2**

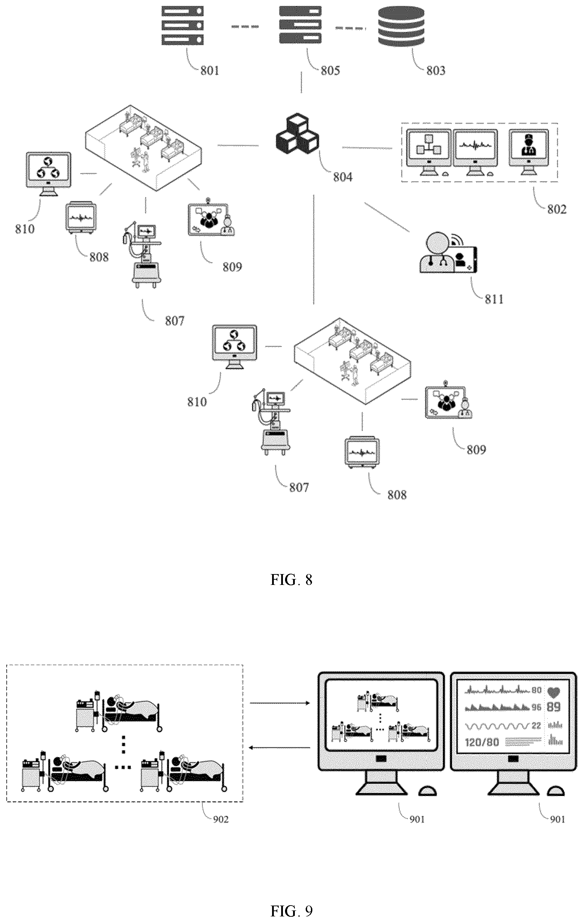

The embodiment of the present application discloses a system of mechanical ventilation therapy data management, as shown in FIG. 8, the system comprises an application software system 801, clinical data terminal devices 802 and a database server 803.

Wherein, the application software system 801 comprises a data acquisition and standardization module, a data cleaning module, a data labeling module and a report generation module.

Specifically, the data acquisition and standardization module is configured to put the patient information and the real-time received mechanical ventilation therapy data into the standard data structure object respectively to form standardized data; the mechanical ventilation therapy data comprises ventilator data.

The data cleansing module is configured to perform data cleansing on the standardized data to obtain a medical data set.

The data labeling module is configured to perform data labeling on the mechanical ventilation therapy data in the medical data set; the data labeling comprises: marking amplitudes, frequencies, and shapes of waveform data, marking measurement values of numerical data, and marking abnormal events.

The report generation module is configured to generate a mechanical ventilation therapy data analysis report based on the medical data set with the data labeling when a report generation instruction is received.

The clinical data terminal devices 802 are configured to issue the report generation instructions, and further configured to receive and display the mechanical ventilation therapy data analysis reports.

The database server 803 is configured to store the standardized data, the medical data sets, and the mechanical ventilation therapy data analysis reports in partitions.

The other data comprised in the mechanical ventilation therapy data, the data labeling process and the mechanical ventilation therapy data analysis report generating process have been described in detail in the embodiment 1, and will not be described again here.

In addition, in the present embodiment, in the data labeling process of the data labeling module, a first aid instruction is issued to the clinical data terminal devices when one or more abnormal events of ventilators stop working, asphyxia, increasing airway pressure, cardiac arrest, tachycardia, bradycardia, ventricular flutter, ventricular fibrillation and hypotension occur, the first aid instruction carries the information about the abnormal events that occur; at this time, the clinical data terminal devices 802 are further configured to receive and respond to the first aid instructions. For example, the clinical data terminal devices 802 can issue an abnormal alarm in respond to the first aid instruction, to draw the attention of the medical staff, and can also display the abnormal events information via the clinical data terminal devices.

In the data labeling process of the data labeling module, a ventilator parameters adjustment instruction is issued to the clinical data terminal devices 802 when one or more abnormal events of patient-ventilator asynchronous event, high airway pressure, low airway pressure, high positive end expiratory pressure, low positive end expiratory pressure, high minute ventilation, low minute ventilation, high tidal volume, low tidal volume, high respiratory rate, low respiratory rate, high oxygen concentration and low oxygen concentration occur, the ventilator parameter adjustment instruction carries the information about the abnormal events that occur; at this time, the clinical data terminal devices 802 are further configured to receive and respond to the ventilator parameters adjustment instructions, the ventilator parameter adjustment instruction can be responded by issuing an abnormal alarm to draw the attention of the medical staff, and can also display the abnormal events information via the clinical data terminal devices 802, and can also provide a suggestion of the ventilator parameter adjustment method for the current abnormal event.

The application software system 801 further comprises a streaming media management module, the streaming media management module is configured to receive streaming media data of the treatment scenes in real time via the bedside audio video devices; the streaming media management module can also manage the operation of the bedside audio video devices, comprising streaming media service management, equipment scheduling, consultation management, ward round management, and live broadcasting, storing, reviewing, and retrieving of streaming media data. At this time, the database server 803 is further configured to store the streaming media data in partitions and associate the streaming media data with the medical data sets and the mechanical ventilation therapy data analysis reports; the clinical data terminal devices 802 are further configured to live broadcast in real time of the waveform data, the numeric data, and the treatment scenes in the mechanical ventilation therapy data of the medical data set as well as the mechanical ventilation therapy data analysis reports of some or all patients in one or more wards.

As shown in FIG. 8, the system of mechanical ventilation therapy data management can further comprise a multi-mode gateway 804 and a master control server 805; wherein the multi-mode gateway is configured to connect the ventilator devices 807, the multi-parameter monitors 808, the bedside audio video devices 809, the clinical information systems 810 and the clinical data terminal devices 802 from different manufacturers in each ward of the hospital through the network; the multi-mode gateway 804 is embedded with a variety of communication protocols, comprising at least Transmission Control Protocol (TCP) / Internet Protocol (IP), User Datagram Protocol (UDP), Recommended Standard 232/485 (RS 232/485), Representational State Transfer Application Programming Interface (RESTful API), Wi-Fi (WIreless FIdelity), Message Queuing Telemetry Transport (MQTT), and Health Level seven (HL7), configured to realize data communication between the ventilator devices 807, the multi-parameter monitors 808, the bedside audio video devices 809, the clinical information systems 810 and the data acquisition and standardization module, and configured to realize the data communication between the application software system 801 and the clinical data terminal devices 802. The master control server 805, embedded with a multi-task operating system and an application software system 801 running on the operating system, configured to control the operation of the application software system 801.

The database server 803, consisting of a plurality of servers to form a database cluster, is configured to store various types of data and files for the system, in addition, is further configured to perform real-time analysis and processing, multi-dimensional retrieval and query, multi-user network operation, and database disaster recovery management. It should be noted that the database server 803 can use a plurality of servers to form the database cluster, wherein one server is set as the master node and the rest are set as sub-nodes, as a copy of the master node, to divert the business pressure of the master node server, when the master node encounters an exception during operation, the sub-nodes can adaptively switch to be the master node, which can improve the security and high availability of the database, and meet the storage of massive mechanical ventilation therapy data, data analysis report files, streaming media data for the system, database cluster can be dynamically expanded or migrated to the cloud more conveniently.

The clinical data terminal devices 802, comprising at least desktop computers and desktop devices with computing capabilities, can be configured to perform data communication with the system and display the mechanical ventilation therapy data and status of the patients; common clinical data terminal devices such as handheld terminal devices 811 held by doctors and nurses, comprising at least laptop computers and smartphones, can be configured to display the mechanical ventilation therapy data and status of the patients.

The bedside audio video devices 809, comprising at least video monitoring devices and audio video mobile carts, can be configured to display the patient treatment scenes on the screen of the clinical data terminal devices.

In the present embodiment, the various modules in the application software system 801 cooperate with each other to jointly complete the functions of data acquisition, data cleaning, data labeling and report generation, which can realize multi-dimensional analysis and comprehensive judgment of the patient's mechanical ventilation therapy process, thereby predicting the disease evolution trend, facilitating doctors to make intervention measures, which effectively improves the quality of mechanical ventilation therapy and work efficiency, reduces labor intensity of doctors and nurses. The screen of the clinical data terminal devices 802 can be a single screen or a combination of screens, the doctors and nurses holding the data terminal devices 811 can perform seamless monitoring and management of the mechanical ventilation therapy patients in any situation.

Preferably, FIG. 9 of the present embodiment shows an implementation example displaying the patient in a visualized way, wherein the clinical data terminal devices 901 can receive the mechanical ventilation therapy data and the streaming media data of the patients 902 in a plurality of wards in real time, dynamically display the waveform data, the numerical data and the treatment scenes of the patients in the plurality of wards in a visualized way to remotely and centrally manage their mechanical ventilation therapy data business of the patients in the plurality of wards.

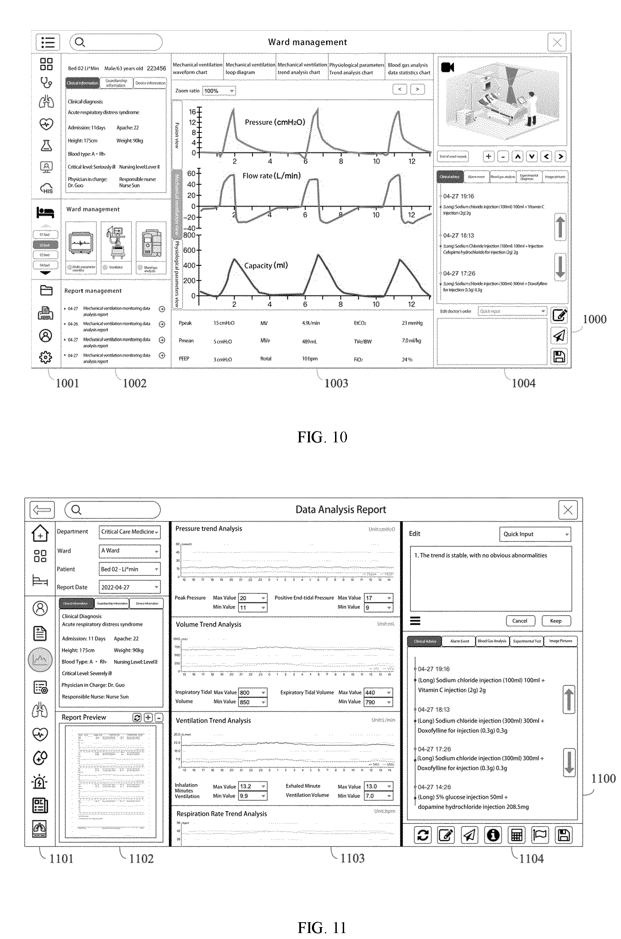

Preferably, a ward management panel 1000 provided by the clinical data terminal device screen as shown in FIG. 10, so as to centrally manage the mechanical ventilation data business of patients in a plurality of wards, can display the patient's mechanical ventilation therapy data and disease evolution trend in a visualized way, observe and analyze a variety of data, events data and treatment scenes, output clinical diagnostic information, as well as medical advice, nursing information and business instructions, wherein the business instructions at least comprise first aid instructions, ventilator parameters adjustment instructions, and generating analysis report instructions. The ward management panel is set up with content window comprising a functional operation area 1001, a ward management area 1002, a patient data management area 1003, and a clinical application management area 1004, as well as functional buttons, which can allow selecting all or a certain patient in a plurality of wards to observe the patient's real-time data, events data, historical data, clinical medical advice and nursing information, and treatment scenes, analyzing the mechanical ventilation therapy, issuing business instructions or medical advice. Specifically, the functional operation area 1001 can be equipped with function buttons, configured to switch between panels of the clinical data terminal devices, and also configured to select a specified patient; the ward management area 1002 can be configured to display and retrieve the patients information, wards information and reports information; the patient data management area 1003 can be configured to display and retrieve the mechanical ventilation therapy data of the patients; the clinical application management area 1004 cab be configured to display and retrieve the patient's streaming media data and mechanical ventilation therapy data, issue business instructions or medical advice.

Preferably, FIG. 11 shows an implementation example of a data analysis report panel 1100 of a clinical data terminal device provided in the embodiment 2 of the present application; the clinical data terminal device screen provides the data analysis report panel to visually display the mechanical ventilation analysis reports of all or a certain patient in a plurality of wards, thereby performing review, network distribution, output printing, and database storage for the mechanical ventilation data analysis reports according to the business instructions or the manual review requests automatically prompted by the medical advice appointment time. The data analysis report panel is set up with content window comprising a functional operation area 1101, a ward management area 1102, an analysis report area 1103, a clinical medical advice area 1104, and functional buttons to centrally manage the mechanical ventilation data analysis report business of a plurality of wards, analyze the reasons for data changes in conjunction with the clinical treatment medical advice information, perform comparative analysis on all the previous mechanical ventilation therapy data analysis reports of a certain patient to determine the disease evolution trend, and trigger the printer to output the mechanical ventilation therapy data analysis reports. Specifically, the function operation area 1101 is equipped with function buttons, configured to switch between data analysis panels of the clinical data terminal devices, and further configured to perform management operations on the reports; the ward management area 1102 is configured to display and retrieve the patients information, the wards information and the reports information, and further configured to select a specified patient or a report; the analysis report area 1103 is configured to display and retrieve the patients' data analysis reports and display them in a visualized way; the clinical medical advice area 1104 is configured to display and retrieve the patients' mechanical ventilation therapy data, edit report contents and trigger print commands.

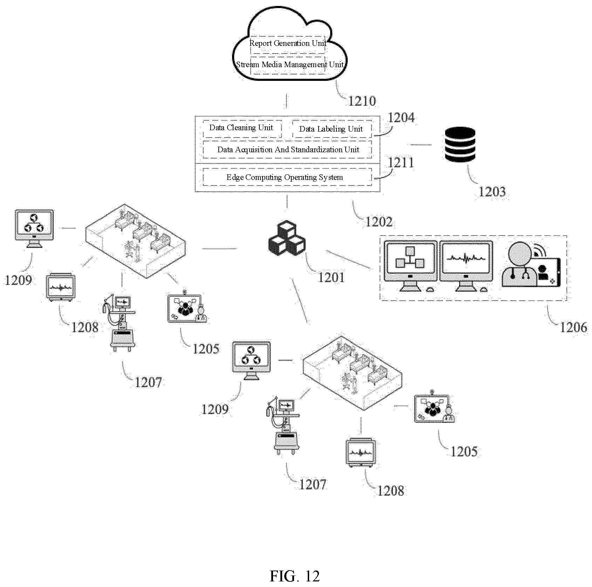

To further extend the scalability and flexibility of the system of mechanical ventilation therapy data management in the present embodiment, for example, the system of mechanical ventilation therapy data management can be deployed under a cloud computing architecture, i.e., each functional module in the system of mechanical ventilation therapy data management is deployed under a cloud computing architecture; or some of the functional modules in the system of mechanical ventilation therapy data management are deployed on a cloud platform, for example, the report generation module and streaming media management module, etc., which are configured to realize various business functions of the system, comprise cloud edge collaborative live broadcast in real time of the waveform data, the numeric data, and the treatment scenes; other functional modules are deployed on edge computing nodes, such as data acquisition and standardization module, data cleaning module, data labeling module, etc., which are configured to perform data cleaning and data standardization at the data source, as well as perform data labeling and events labeling, providing support for cloud edge collaboration, cloud platform data storage management, fast multi-dimensional data retrieval, and data updating. As shown in FIG. 12, the system comprises a multi-mode gateway 1201, a master control server 1202, a database server 1203, an application software system 1204, bedside audio video devices 1205, clinical data terminal devices 1206; the multi-mode gateway 1201 connects the bedside audio video devices 1205, the ventilators 1207, the multi-parameter monitors 1208, the clinical information systems 1209 and the clinical data terminal devices 1206 from different manufacturers in each ward of the hospital through the network, and establishes a connection with the cloud platform 1210.

The system of mechanical ventilation therapy data management in the present embodiment can also connect to a third-party medical data service, entrusting the real-time monitoring, data analysis and processing of the complex and tedious mechanical ventilation therapy data to a third-party for management, so that the doctors and nurses are able to focus on the clinical medical issues of the mechanical ventilation therapy, to further reduce the work pressure of the doctors and nurses, and to improve the quality of the mechanical ventilation therapy and the work efficiency.

The above method and system embodiments improve the existing clinical application method of the mechanical ventilation therapy data, doctors and nurses can remotely and centrally manage the patients in a plurality of wards, quickly grasp comprehensive mechanical ventilation data and the disease evolution trend, which solves the difficulties caused by huge multi-source heterogeneous data to the clinical application, reduces the work pressure of doctors and nurses, improves the quality of mechanical ventilation therapy, and has wide applicability.

The present embodiment can reduce hardware costs and business pressure, performs data preprocessing at the data source, reduce the delay of network data communication and cloud platform processing effectively, can flexibly determine the scale of user data storage, for example, limiting the data files to be saved for a period of three months, reduce the pressure and cost of storing the mechanical ventilation therapy data. When there is a malfunction in the external network, the edge computing nodes can complete the processing of the mechanical ventilation therapy data analysis reports independently.

A person skilled in the art can understand that all or part of the process of implementing the methods of the above embodiments may be implemented by related hardware according to an instruction from a computer program, and the program may be stored in a computer-readable storage medium, wherein the computer-readable storage medium is a magnetic disc, an optical disc, a read-only memory, a random access memory and so on.

The above are merely preferable particular embodiments of the present application, and the protection scope of the present application is not limited thereto.

## Claims

1. A method of mechanical ventilation therapy data management, wherein the method comprises:

putting patient information and real-time received mechanical ventilation therapy data into a standard data structure object (201) respectively, to form standardized data (S1); the mechanical ventilation therapy data comprises ventilator data; wherein the mechanical ventilation therapy data further comprises one or more of the following data: physiological parameters data, blood gas analysis data, laboratory diagnostic data, imaging data, clinical diagnosis and treatment information, and the ID of the device for outputting the mechanical ventilation therapy data; wherein, the physiological parameters data is output by multi-parameter monitors (808, 1208); the patient information, the blood gas analysis data, the laboratory diagnostic data, the imaging data, and the clinical diagnosis and treatment information are all output by a clinical information system (810, 1209); the mechanical ventilation data is output by ventilator devices (807);
performing data cleaning on the standardized data to obtain a medical data set (S2);
performing data labeling on the mechanical ventilation therapy data in the medical data set (S3); the data labeling comprises: marking amplitudes, frequencies, and shapes of waveform data, marking measurement values of numerical data, and marking abnormal events;
when a report generation instruction is received, generating a mechanical ventilation therapy data analysis report based on the medical data set with the data labeling (S4);
wherein in the mechanical ventilation data, the waveform data comprises: mechanical ventilation pressure waveform data, volume waveform data, flow waveform data and end-tidal carbon dioxide waveform data; the numerical data comprises: airway resistance, work of breathing, compliance, ventilation parameters and respiratory rate;
in the data labeling process for the mechanical ventilation data,
the shapes of the labeled waveform data comprise: beginning of inspiratory phase, inspiratory phase, end of inspiratory phase, beginning of expiratory phase, expiratory phase, end of expiratory phase, peak airway pressure, and peak expiratory flow rate of each mechanical ventilation waveform, as well as inspiratory baseline, expiratory plateau height, beginning and end of expiratory plateau of the end-tidal carbon dioxide waveform;
the labeled abnormal events comprise: patient-ventilator asynchronous event, high/low airway pressure, high/-low positive end-expiratory pressure, high/low minute ventilation, high/low oxygen concentration, high/low respiratory rate, high/low partial pressure of end-tidal carbon dioxide, high/low tidal volume, ventilators stop working, asphyxia and increasing airway pressure;

wherein, the method determines the patient-ventilator asynchronous even based on a neural network model, by executing the following steps:

constructing model inputs $T = \langle S_1, \cdots, S_c, \cdots, S_C \rangle$ based on a pressure-time curve of the mechanical ventilation pressure waveform data, a volume-time curve of the mechanical ventilation volume waveform data, and a flow-time curve of the mechanical ventilation flow waveform data; wherein $S_c$ represents an input vector of a c-th sampling point, C represents a total number of sampling points processed by the neural network model in a single run, that is, a length of the model inputs; $S_c = \left( S_c^1, S_c^2, S_c^3 \right)$, $S_c^1$, $S_c^2$, $S_c^3$ represent values of flow rate, pressure, and volume at the c-th sampling point respectively;

performing two times sliding window processing for feature enrichment to obtain time series after sliding window processing; wherein

in a first sliding window processing, executing: performing sliding window processing to the model input $T$ with b/2 as sliding window step, $b$ represents a width of a first sliding window; after the first sliding window processing, model input length is changed from C to C', values range of c is changed to [1, C'];

$$C' = \frac{2*C - b}{b}$$ ;

after the first sliding window processing, based on a class $l$ waveform data in all sampling points within a c-th sliding window, calculating a Euclidean distance $NOR_c^l$ of the class $l$ waveform data in Euclidean matrix vector, that is, $NOR_c^l = \sqrt{\left( S_c^l \right)^2 + \left( S_{c+1}^l \right)^2 + \cdots + \left( S_{c+b-1}^l \right)^2}$ , $l$ takes 1, 2, 3 respectively; finally, obtaining Euclidean matrix vector $NOR_c = \left( NOR_c^1, NOR_c^2, NOR_c^3 \right)$ ; based on the class $l$ waveform data in all sampling points within the c-th sliding window, calculating a distance difference $DON_c^l$ of the class $l$ waveform data in distance difference vector, that is, $$DON_c^l = NOR_{c+1}^l - NOR_c^l$$ ; finally, the distance difference vector is obtained and represented as $DON_c = \left( DON_c^1, DON_c^2, DON_c^3 \right)$ ; therefore, time series after the first sliding window processing is: $T' = \langle G_1, \cdots, G_c, \cdots, G_C \rangle$,

wherein, $G_c$ $\langle NOR_c, DON_c \rangle$; in a second sliding window processing, executing: performing sliding window processing to the time series $T'$ again with f/2 as sliding window step, $f$ represents a width of a second time sliding window; after the second time sliding window processing, model input length is changed from C' to C", values range of c is changed to [1, C"]; $C'' = \frac{2*C' - f}{f}$ ; during the second sliding window processing, obtaining derived features of all Euclidean matrix vectors and distance difference vectors within the second time sliding window, to obtain time series after the second time sliding window processing;

inputting the time series after sliding window processing into the neural network model, after processing, the neural network model outputs normal event as well as ventilator abnormal events, comprising type and occurrence time of the patient-ventilator asynchronous event.

2. The method of mechanical ventilation therapy data management according to claim 1, wherein in the physiological parameters data, the waveform data comprises: electrocardiogram waveform data, respiratory waveform data, photoplethysmogram waveform data and invasive arterial pressure waveform data; the numerical data comprises:

heart rate, blood pressure, blood oxygen saturation and body temperature;

in the data labeling process for the physiological parameters data,
the shapes of the labeled electrocardiogram waveform data comprise: P wave, QRS complex, T wave, ST segment, PR interval, RR interval and QT interval of each ECG cycle waveform;
the shapes of the labeled respiratory waveform data comprise: peaks and valleys of each respiratory cycle waveform;
the shapes of the labeled photoplethysmogram waveform data comprise: peaks and valleys of each pulse cycle waveform;
the shapes of the labeled invasive arterial pressure waveform data comprise: peaks and valleys of each arterial systolic-diastolic cycle waveform;
the labeled abnormal events comprise: tachycardia, bradycardia, cardiac arrest, atrial flutter, atrial fibrillation, ventricular flutter, ventricular fibrillation, atrioventricular block, ST-segment elevation/depression, high/low blood pressure, high/low body temperature and low blood oxygen saturation.

3. The method of mechanical ventilation therapy data management according to claim 2, wherein, in the blood gas analysis data, the numerical data comprises: partial pressure of oxygen, partial pressure of carbon dioxide, total carbon dioxide, potassium ion concentration, sodium ion concentration, bicarbonate ion concentration and pH value;

in the data labeling process for the blood gas analysis data,
the labeled abnormal events comprise: high/low partial pressure of oxygen, high/low partial pressure of carbon dioxide, high/low total carbon dioxide, high/low potassium ion concentration, high/low sodium ion concentration and high/low pH value.

4. The method of mechanical ventilation therapy data management according to claim 3, wherein in the laboratory diagnostic data, the numerical data comprises: white blood cell count, red blood cell count, platelet count, hemoglobin, hematocrit, blood glucose, prothrombin time and activated partial thrombin time;

in the data labeling process for the laboratory diagnostic data,
the labeled abnormal events comprise: high/low white blood cell count, high/low red blood cell count, high/low hemoglobin, high/low hematocrit, high/low blood glucose, high/low platelet count, long/short prothrombin time, and long/short activated partial thrombin time.

5. The method of mechanical ventilation therapy data management according to claim 4, wherein the reporting framework of the mechanical ventilation therapy data analysis report comprises a report overview page (400) and a report analysis page; wherein the report overview page (400) is divided into a basic information area (401), a mechanical ventilation data area (402), and an analysis conclusion area (403);

the basic information area (401) comprises at least the patient information and key time point information; and further comprises the following options: the clinical diagnosis information, the ID of the device for outputting the mechanical ventilation therapy data as well as mechanical ventilation mode and setting information;
the mechanical ventilation data area (402) comprises at least respiratory analysis results, patient-ventilator asynchronous events analysis results, airway resistance change analysis results, compliance change analysis results, and lung injury early warning analysis results; each of the analysis results of the mechanical ventilation data area (402) is obtained based on the mechanical ventilation data and its data labeling; the respiratory analysis results comprise a rapid shallow breathing index;
the analysis conclusion area (403) is configured to display the analysis descriptions and conclusions of the mechanical ventilation therapy data;
the report analysis page is configured to display a detailed analysis report of one or more mechanical ventilation therapy data separately in graphical form.

6. The method of mechanical ventilation therapy data management according to claim 5, wherein the report analysis page comprises a trend graph page (500) and a data page;

the trend graph page (500) comprises:

trend analysis areas of pressure, volume, and ventilation volume of the mechanical ventilation data;
a trend analysis area of a respiratory frequency (504);

a trend analysis area of an inhaled oxygen concentration (505); and

a trend analysis description and conclusion area (506) is configured to synthesize the trend direction of each trend analysis area, to provide trend analysis descriptions and corresponding conclusions;

the contents of each trend analysis area in the trend graph page (500) are obtained based on the mechanical ventilation data and its data labeling;

the data page comprises at least:

a mechanical ventilation setting parameters data sub-page (610) comprises: a mechanical ventilation setting parameters record sheet (611), ventilation modes of mechanical ventilation and a change trend graph of setting parameters (612);

a mechanical ventilation monitoring parameters data sub-page (620) comprises a mechanical ventilation monitoring parameters record sheet (621);

the contents of the mechanical ventilation setting parameters data sub-page (610) and the mechanical ventilation monitoring parameters data sub-page (620) are obtained based on the mechanical ventilation data and its data labeling;

the data page further comprises the following optional data sub-pages:

a 24-hour physiological parameters data sub-page (630) comprises a 24-hour vital signs monitoring record sheet and a heart rate/respiratory rate/blood pressure trend graph; the contents of the 24-hour physiological parameters data sub-page (630) are obtained based on the physiological parameters data and its data labeling;

a 24-hour blood gas analysis and laboratory diagnostic data sub-page (640) comprises a 24-hour blood gas analysis monitoring record sheet, a partial pressure of oxygen/partial pressure of carbon dioxide/-bicarbonate trend graph, and a laboratory diagnostic data sheet;

the contents of the 24-hour blood gas analysis and laboratory diagnostic data sub-page (640) are obtained based on the blood gas analysis data, the laboratory diagnostic data, and their data labeling;

an alarm events data sub-page (650) comprises: an alarm events record sheet, a 24-hour alarm events distribution diagram, and an alarm events comprehensive analysis sheet;

the alarm events data sub-page (650) is obtained based on the abnormal events labeled by the mechanical ventilation therapy data;

an extended image data sub-page (660) is configured to display the image data.

7. The method of mechanical ventilation therapy data management according to claim 6, wherein, the report analysis page further comprises a graphic page; the graphic page is divided into a mechanical ventilation waveform data area (701), a mechanical ventilation loop chart area (703), and a physiological parameters waveform data area (702); wherein

the mechanical ventilation waveform data area (701) comprises waveform graphs of continuous normal mechanical ventilation data and waveform graphs of abnormal mechanical ventilation events data;

the mechanical ventilation loop chart area (703) comprises a pressure-volume loop chart and a flow-volume loop chart drawn by the combination of the mechanical ventilation pressure, volume, and flow data;

the contents of the mechanical ventilation waveform data area (701) and the mechanical ventilation loop chart area (703) are obtained based on the mechanical ventilation data and its data labeling;

the physiological parameters waveform data area (702) comprises waveform graphs of continuous physiological parameters data synchronized with the mechanical ventilation data waveform graphs;

the content of the physiological parameters waveform data area (702) is obtained based on the physiological parameters data and its data labeling.

8. The method of mechanical ventilation therapy data management according to any of claims 5-7, wherein, the report generation instruction is a customized report generation instruction;

the customized report generation instruction comprises one or more customized parameters, is configured to generate the report overview page (400), and the report analysis page matching the customized parameters;

the customized parameters comprise the trend graph page (500), the data page, or the graphic page.

9. The method of mechanical ventilation therapy data management according to any of claims 5-7, wherein the method further comprises:

in the data labeling process, a first aid instruction is issued when one or more abnormal events of ventilators stop working, asphyxia, increasing airway pressure, cardiac arrest, tachycardia, bradycardia, ventricular flutter, ventricular fibrillation and hypotension occur, the first aid instruction carries the information about the abnormal events that occur; or

in the data labeling process, a ventilator parameters adjustment instruction is issued when one or more abnormal events of patient-ventilator asynchronous event, high airway pressure, low airway pressure, high positive end expiratory pressure, low positive end expiratory pressure, high minute ventilation, low minute ventilation, high tidal volume, low tidal volume, high respiratory rate, low respiratory rate, high oxygen concentration and low oxygen concentration occur, wherein the ventilator parameters adjustment instruction carries the information about the abnormal events that occur.

10. The method of mechanical ventilation therapy data management according to any of claims 1-4, wherein, in the standard data structure object (201), a corresponding data structure object is configured for each item of the mechanical ventilation therapy data and the patient information respectively;

each mechanical ventilation therapy data decoupled and the patient information are placed into the corresponding data structure object to form standardized data.

11. A system of mechanical ventilation therapy data management, wherein, the system comprises an application software system (801, 1204), clinical data terminal devices (802, 901, 1206) and a database server (803, 1203); wherein,
the application software system (801, 1204) comprises:

a data acquisition and standardization module, configured to put the patient information and the real-time received mechanical ventilation therapy data into the standard data structure object (201) respectively to form standardized data; the mechanical ventilation therapy data comprises ventilator data; wherein the mechanical ventilation therapy data further comprises one or more of the following data: physiological parameters data, blood gas analysis data, laboratory diagnostic data, imaging data, clinical diagnosis and treatment information, and the ID of the device for outputting the mechanical ventilation therapy data; wherein, the physiological parameters data is output by multi-parameter monitors (808, 1208); the patient information, the blood gas analysis data, the laboratory diagnostic data, the imaging data, and the clinical diagnosis and treatment information are all output by a clinical information system (810, 1209); the mechanical ventilation data is output by ventilator devices (807);
a data cleaning module is configured to perform data cleaning on the standardized data to obtain a medical data set;
a data labeling module is configured to perform data labeling on the mechanical ventilation therapy data in the medical data set;
the data labeling comprises: marking amplitudes, frequencies, and shapes of waveform data, marking measurement values of numerical data, and marking abnormal events;
a report generation module is configured to generate a mechanical ventilation therapy data analysis report based on the medical data set with the data labeling when a report generation instruction is received;
the clinical data terminal devices (802, 901, 1206) are configured to issue the report generation instructions, and further configured to receive and display the mechanical ventilation therapy data analysis reports;
the database server (803, 1203) is configured to store the standardized data, the medical data sets, and the mechanical ventilation therapy data analysis reports in partitions ;
wherein in the mechanical ventilation data, the waveform data comprises: mechanical ventilation pressure waveform data, volume waveform data, flow waveform data and end-tidal carbon dioxide waveform data; the numerical data comprises: airway resistance, work of breathing, compliance, ventilation parameters and respiratory rate;
in the data labeling process for the mechanical ventilation data,
the shapes of the labeled waveform data comprise: beginning of inspiratory phase, inspiratory phase, end of inspiratory phase, beginning of expiratory phase, expiratory phase, end of expiratory phase, peak airway pressure, and peak expiratory flow rate of each mechanical ventilation waveform, as well as inspiratory baseline, expiratory plateau height, beginning and end of expiratory plateau of the end-tidal carbon dioxide waveform;
the labeled abnormal events comprise: patient-ventilator asynchronous event, high/low airway pressure, high/-low positive end-expiratory pressure, high/low minute ventilation, high/low oxygen concentration, high/low respiratory rate, high/low partial pressure of end-tidal carbon dioxide, high/low tidal volume, ventilators stop working, asphyxia and increasing airway pressure;
wherein, the system is configured to determine the patient-ventilator asynchronous even based on a neural network model, by executing the following steps:

constructing model inputs $T = \langle S_1, \cdots, S_c, \cdots, S_C \rangle$ based on a pressure-time curve of the mechanical ventilation pressure waveform data, a volume-time curve of the mechanical ventilation volume waveform data, a flow-time curve of the mechanical ventilation flow waveform data; wherein $S_c$ represents an input vector of a c-th sampling point, C represents a total number of sampling points processed by the neural network model in a single run, that is, a length of the model inputs; $S_c = \left( S_c^1, S_c^2, S_c^3 \right)$, $S_c^1$, $S_c^2$, $S_c^3$ represent values of flow rate, pressure, and volume at the c-th sampling point respectively;

performing two times sliding window processing for feature enrichment to obtain time series after sliding window processing; wherein

in a first sliding window processing, executing: performing sliding window processing to the model input $T$ with b/2 as sliding window step, $b$ represents a width of a first sliding window; after the first sliding window processing, model input length is changed from $C$ to $C'$, values range of c is changed to [1, $C'$];

$$C' = \frac{2*C-b}{b} \ ;$$

after the first sliding window processing, based on a class $l$ waveform data in all sampling points within a c-th sliding window, calculating a Euclidean distance $NOR_c^l$ of the class $l$ waveform data in Euclidean matrix vector, that is, $NOR_c^l = \sqrt{\left( S_c^l \right)^2 + \left( S_{c+1}^l \right)^2 + \cdots + \left( S_{c+b-1}^l \right)^2}$ , $l$ takes 1, 2, 3 respectively; finally, obtaining Euclidean matrix vector $NOR_c = \left( NOR_c^1, NOR_c^2, NOR_c^3 \right)$ ; based on the class $l$ waveform data in all sampling points within the c-th sliding window, calculating a distance difference $DON_c^l$ of the class $l$ waveform data in distance difference vector, that is, $$DON_c^l = NOR_{c+1}^l - NOR_c^l$$ ; finally, the distance difference vector is obtained and represented as $DON_c = \left( DON_c^1, DON_c^2, DON_c^3 \right)$ ; therefore, time series after the first sliding window processing is: $T' = \langle G_1, \cdots, G_c, \cdots, G_C \rangle$, wherein, $G_c = \langle NOR_c, DON_c \rangle$ ; in a second sliding window processing, executing: performing sliding window processing to the time series $T$ again with f/2 as sliding window step, $f$ represents a width of a second time sliding window; after the second time sliding window processing, model input length is changed from $C'$ to $C''$, values range of c is changed to [1, $C''$]; $C'' = \frac{2*C'-f}{f}$ ; during the second sliding window processing, obtaining derived features of all Euclidean matrix vectors and distance difference vectors within the second time sliding window, to obtain time series after the second time sliding window processing;

inputting the time series after sliding window processing into the neural network model, after processing, the neural network model outputs normal event as well as ventilator abnormal events, comprising type and occurrence time of the patient-ventilator asynchronous event.

12. The system of mechanical ventilation therapy data management according to claim 11, wherein the application software system (801, 1204) further comprises a streaming media management module, the streaming media management module is configured to receive streaming media data of the treatment scenes in real time; the streaming media data is output by bedside audio video devices (809, 1205);

the database server (803, 1203) is further configured to store the streaming media data in partitions and associate the streaming media data with the medical data sets and the mechanical ventilation therapy data analysis reports; the clinical data terminal devices (802, 901, 1206) are further configured to live broadcast in real time of the waveform data, the numeric data, and the treatment scenes in the mechanical ventilation therapy data of the medical data set as well as the mechanical ventilation therapy data analysis reports of some or all patients in one or more wards.

13. The system of mechanical ventilation therapy data management according to claim 11, wherein the system further comprises a multi-mode gateway (804, 1201) and a master control server (805, 1205); wherein

the multi-mode gateway (804, 1201) is configured to connect ventilator devices (807), multi-parameter monitors (808, 1208), bedside audio video devices (809, 1205), clinical information systems (810, 1209) and clinical data terminal devices (802, 901, 1206) from different manufacturers in each ward of the hospital through the network; the multi-mode gateway (804, 1201) is embedded with a variety of communication protocols to realize the data communication between the ventilator devices (807), the multi-parameter monitors (808, 1208), the bedside audio video devices (809, 1205), the clinical information systems (810, 1209) and the data acquisition and standardization module, and configured to realize the data communication between the application software system (801, 1204) and the clinical data terminal devices (802, 901, 1206) where the patients are located; the master control server (805, 1205) is embedded with a multi-task operating system and an application software system (801, 1204) running on the multi-task operating system, is configured to control the operation of the application software system (801, 1204).

**Patentansprüche**

1. Ein Verfahren zur Datenverwaltung in der mechanischen Beatmungstherapie, wobei das Verfahren umfasst:

Einfügen von Patienteninformationen und in Echtzeit empfangenen Daten zur mechanischen Beatmungstherapie jeweils in ein Standarddatenstrukturobjekt (201), um standardisierte Daten (S1) zu bilden; die Daten zur mechanischen Beatmungstherapie Beatmungsdaten umfassen; wobei die Daten zur mechanischen Beatmungstherapie ferner einen oder mehrere der folgenden Datentypen umfassen: Daten physiologischer Parameter, Blutgasanalysedaten, Labordiagnosedaten, Bildgebungsdaten, klinische Diagnose- und Behandlungsinformationen sowie die Geräte-ID zur Ausgabe der Daten zur mechanischen Beatmungstherapie; wobei die Daten physiologischer Parameter von Mehrparameter-Monitoren (808, 1208) ausgegeben werden; die Patienteninformationen, die Blutgasanalysedaten, die Labordiagnosedaten, die Bildgebungsdaten sowie die klinische Diagnose- und Behandlungsinformationen jeweils von einem klinischen Informationssystem (810, 1209) ausgegeben werden; die mechanischen Beatmungsdaten von Beatmungsgeräten (807) ausgegeben werden; Datenbereinigung der standardisierten Daten, um einen medizinischen Datensatz (S2) zu erhalten; Durchführen einer Datenkennzeichnung für die Daten zur mechanischen Beatmungstherapie in dem medizinischen Datensatz (S3); die Datenkennzeichnung umfasst: Markieren der Amplituden, Frequenzen und Formen von Wellenformdaten, Markieren der Messwerte numerischer Daten und Markieren abnormaler Ereignisse; bei Empfang eines Befehls zur Berichtserstellung, Generieren eines Analyseberichts für Daten zur mechanischen Beatmungstherapie auf Basis des medizinischen Datensatzes (S4) mit Datenkennzeichnung; wobei in den mechanischen Beatmungsdaten, die Wellenformdaten umfassen: mechanische Beatmungsdruck-Wellenformdaten, Volumenwellenformdaten, Flusswellenformdaten und endtidale Kohlendioxidwellenformdaten; numerische Daten umfassen: Atemwegswiderstand, Atemarbeit, Compliance, Beatmungsparameter und Atemfrequenz; bei der Datenkennzeichnung der mechanischen Beatmungsdaten, die Formen der markierten Wellenformdaten umfassen: Beginn der Inspirationsphase, Inspirationsphase, Ende der Inspirationsphase, Beginn der Exspirationsphase, Exspirationsphase, Ende der Exspirationsphase, Atemwegsspitzendruck und exspiratorischer Spitzenfluss jeder mechanischen Beatmungswelle sowie Inspirationsbasislinie, Höhe des Exspirationsplateaus, Beginn und Ende des Exspirationsplateaus der endtidalen Kohlendioxidwelle; die markierten abnormalen Ereignisse umfassen: Patient-Beatmungsgerät-Asynchronie, hoher/niedriger Atemwegsdruck, hoher/niedriger positiver endexspiratorischer Druck, hohes/niedriges Minutenvolumen, hohe/niedrige Sauerstoffkonzentration, hohe/niedrige Atemfrequenz, hoher/niedriger endtidaler Kohlendioxidpartialdruck, hohes/niedriges Atemzugvolumen, Ausfall des Beatmungsgeräts, Erstickung und ansteigender Atemwegsdruck; wobei das Verfahren die Patient-Beatmungsgerät-Asynchronie auf Basis eines neuronalen Netzwerkmodells erkennt, indem folgende Schritte ausgeführt werden:

Aufbauen der Modelleingabe $T = \langle S_1, \cdots, S_c, \cdots, S_C \rangle$ auf Basis der Druck-Zeit-Kurve der mechanischen Beatmungsdruck-Wellenformdaten, der Volumen-Zeit-Kurve der mechanischen Beatmungsvolumen-Wellenformdaten und der Fluss-Zeit-Kurve der mechanischen Beatmungsfluss-Wellenformdaten; wobei $S_c$ der Eingabevektor des c-ten Abtastpunkts ist, C die Gesamtanzahl der Abtastpunkte darstellt, die das neuronale Netzwerkmodell einmal verarbeitet, d. h. die Modelleingabelänge;

$$S_c = (S_c^1, S_c^2, S_c^3), \qquad S_c^1, S_c^2, S_c^3$$

, jeweils die Werte für Fluss, Druck und Volumen des c-ten Abtastpunkts darstellen;

Durchführen von zwei Gleitfensterverarbeitungen zur Merkmalsanreicherung, um eine zeitliche Reihe nach Gleitfensterverarbeitung zu erhalten; wobei

bei der ersten Gleitfensterverarbeitung, Ausführung: Durchführen einer Gleitfensterverarbeitung der Modelleingabe $T$ mit der Schrittweite b/2, wobei b die erste Gleitfensterbreite darstellt; nach der ersten Gleitfensterverarbeitung sich die Modelleingabelänge von $C$ auf $C'$ ändert, der Wertebereich von c [1, $C'$] wird;

$$C' = \frac{2*C-b}{b};$$

nach der ersten Gleitfensterverarbeitung auf Basis der $l$-ten Wellenformdaten aller Abtastpunkte im c-ten Gleitfenster die euklidische Distanz der $l$-ten Wellenformdaten im euklidischen Matrixvektor $NOR_c^l$

berechnet wird, d. h. $NOR_c^l = \sqrt{\left(S_c^l\right)^2 + \left(S_{c+1}^l\right)^2 + \cdots + \left(S_{c+b-1}^l\right)^2}$ ; $l$ jeweils 1, 2, 3 annimmt; schließlich der euklidische Matrixvektor $NOR_c = (NOR_c^1, NOR_c^2, NOR_c^3)$ erhalten wird; auf Basis der $l$-ten Wellenformdaten aller Abtastpunkte im c-ten Gleitfenster der Distanzunterschied der $l$-ten Wellenformdaten im Distanzdifferenzvektor $DON_c^l$ berechnet wird, d. h.

$$DON_c^l = NOR_{c+1}^l - NOR_c^l \quad; \qquad \text{schließlich} \qquad \text{der} \qquad \text{Distanzdifferenzvektor} \qquad \text{als}$$

$$DON_c = (DON_c^1, DON_c^2, DON_c^3)$$ dargestellt wird; daher die zeitliche Reihe nach der ersten Gleitfensterverarbeitung $T' = \langle G_1, \cdots, G_c, \cdots, G_C \rangle$ ist, wobei $G_c = \langle NOR_c, DON_c \rangle$;

bei der zweiten Gleitfensterverarbeitung, Ausführung: Durchführen einer erneuten Gleitfensterverarbeitung der zeitlichen Reihe $T'$ mit der Schrittweite f/2, wobei f die zweite Gleitfensterbreite darstellt; nach der zweiten Gleitfensterverarbeitung sich die Modelleingabelänge von C' auf C " ändert, der Wertebereich von c [1, $C''$]

$$C'' = \frac{2*C'-f}{f}$$

wird; ; während der zweiten Gleitfensterverarbeitung abgeleitete Merkmale aller euklidischen Matrixvektoren und Distanzdifferenzvektoren im zweiten Gleitfenster ermittelt werden, um die zeitliche Reihe nach der zweiten Gleitfensterverarbeitung zu erhalten;

Eingeben der zeitlichen Reihe nach Gleitfensterverarbeitung in das neuronale Netzwerkmodell, das neuronale Netzwerkmodell nach Verarbeitung normale Ereignisse und abnormale Beatmungsgeräteereignisse ausgibt, einschließlich Art und Zeitpunkt der Patient-Beatmungsgerät-Asynchronie.

2. Das Verfahren zur Datenverwaltung in der mechanischen Beatmungstherapie nach Anspruch 1, wobei bei den Daten physiologischer Parameter die Wellenformdaten umfassen: Elektrokardiogramm-Wellenformdaten, Atemwellenformdaten, photoplethysmographische Wellenformdaten und invasive arterielle Druckwellenformdaten; numerische Daten umfassen: Herzfrequenz, Blutdruck, Blutsauerstoffsättigung und Körpertemperatur;

bei der Datenkennzeichnung der Daten physiologischer Parameter,
die markierten Formen der Elektrokardiogramm-Wellenformdaten umfassen: P-Welle, QRS-Komplex, T-Welle, ST-Strecke, PR-Intervall, RR-Intervall und QT-Intervall jeder EKG-Zykluswelle;
die markierten Formen der Atemwellenformdaten umfassen: Scheitel und Tal jeder Atemzykluswelle;

die markierten Formen der photoplethysmographischen Wellenformdaten umfassen: Scheitel und Tal jeder Pulszykluswelle;

die markierten Formen der invasiven arteriellen Druckwellenformdaten umfassen: Scheitel und Tal jeder arteriellen systolisch-diastolischen Zykluswelle;

die markierten abnormalen Ereignisse umfassen: Tachykardie, Bradykardie, Herzstillstand, Vorhofflattern, Vorhofflimmern, Kammerflattern, Kammerflimmern, atrioventrikulärer Block, ST-Strecken-Hebung/Senkung, hoher/niedriger Blutdruck, hohe/niedrige Körpertemperatur und niedrige Blutsauerstoffsättigung.

3. Das Verfahren zur Datenverwaltung in der mechanischen Beatmungstherapie nach Anspruch 2, wobei die numerischen Daten in den Blutgasanalysedaten umfassen: Sauerstoffpartialdruck, Kohlendioxidpartialdruck, Gesamtkohlendioxid, Kaliumionenkonzentration, Natriumionenkonzentration, Bicarbonationenkonzentration und pH-Wert;

bei der Datenkennzeichnung der Blutgasanalysedaten,
die markierten abnormalen Ereignisse umfassen: hoher/niedriger Sauerstoffpartialdruck, hoher/niedriger Kohlendioxidpartialdruck, hohes/niedriges Gesamtkohlendioxid, hohe/niedrige Kaliumionenkonzentration, hohe/niedrige Natriumionenkonzentration und hoher/niedriger pH-Wert.

4. Das Verfahren zur Datenverwaltung in der mechanischen Beatmungstherapie nach Anspruch 3, wobei die numerischen Daten in den Labordiagnosedaten umfassen: Leukozytenzahl, Erythrozytenzahl, Thrombozytenzahl, Hämoglobin, Hämatokrit, Blutzucker, Prothrombinzeit und aktivierte partielle Thromboplastinzeit;

bei der Datenkennzeichnung der Labordiagnosedaten,
die markierten abnormalen Ereignisse umfassen: hohe/niedrige Leukozytenzahl, hohe/niedrige Erythrozytenzahl, hohes/niedriges Hämoglobin, hoher/niedriger Hämatokrit, hoher/niedriger Blutzucker, hohe/niedrige Thrombozytenzahl, lange/kurze Prothrombinzeit und lange/kurze aktivierte partielle Thromboplastinzeit.

5. Das Verfahren zur Datenverwaltung in der mechanischen Beatmungstherapie nach Anspruch 4, wobei der Berichtsrahmen des Analyseberichts für Daten zur mechanischen Beatmungstherapie eine Übersichtsseite (400) und eine Analyseseite umfasst; wobei die Übersichtsseite (400) unterteilt in einen Basisinformationsbereich (401), einen Beatmungsdatenbereich (402) und einen Analyseergebnisbereich (403) ist;

der Basisinformationsbereich (401) mindestens umfasst: Patienteninformationen und wichtige Zeitstempelinformationen; ferner folgende optionale Angaben umfasst: klinische Diagnoseinformationen, Geräte-ID zur Ausgabe der Daten zur mechanischen Beatmungstherapie sowie Informationen zum Beatmungsmodus und zu Einstellungen;
der Beatmungsdatenbereich (402) mindestens umfasst: Atemanalyseergebnisse, Ergebnisse der Analyse von Patient-Beatmungsgerät-Asynchronie, Ergebnisse der Analyse von Atemwegswiderstandsänderungen, Ergebnisse der Analyse von Compliance-Änderungen und Ergebnisse der Lungenverletzungsvorwarnung; jedes der Analyseergebnisse des Beatmungsdatenbereich (402) auf Basis mechanischer Beatmungsdaten und deren Datenkennzeichnung ermittelt werden; die Atemanalyseergebnisse einen Index für schnelle flache Atmung umfassen;
der Analyseergebnisbereich (403) zur Anzeige von Analysebeschreibungen und Schlussfolgerungen der Daten zur mechanischen Beatmungstherapie dient;
die Analyseseite zur getrennten Anzeige detaillierter Analyseberichte für einen oder mehrere Datentypen der mechanischen Beatmungstherapie in Diagrammform dient.

6. Das Verfahren zur Datenverwaltung in der mechanischen Beatmungstherapie nach Anspruch 5, wobei die Analyseseite eine Trenddiagrammseite (500) und eine Datenseite umfasst; die Trenddiagrammseite (500) umfasst:

Trendanalysebereich für Druck, Volumen und Ventilationsvolumen der mechanischen Beatmungsdaten;
einen Trendanalysebereich für Atemfrequenz (504);
einen Trendanalysebereich für eingeleitete Sauerstoffkonzentration (505); und
einen Trendanalyse-Beschreibungs- und Schlussfolgerungsbereich (506), der zur Zusammenfassung der Trendrichtungen aller Trendanalysebereiche und zur Angabe von Trendanalysebeschreibungen und entsprechenden Schlussfolgerungen dient;
die Inhalte der einzelnen Trendanalysebereiche in der Trenddiagrammseite (500) auf Basis mechanischer Beatmungsdaten und deren Datenkennzeichnung ermittelt werden;
die Datenseite mindestens umfasst:

eine Unterseite für Einstellparameterdaten der mechanischen Beatmung (610) umfasst: eine Aufzeichnungstabelle für Einstellparameter der mechanischen Beatmung (611), ein Trenddiagramm für Änderungen des Beatmungsmodus und der Einstellparameter der mechanischen Beatmung (612);

eine Unterseite für Überwachungsparameterdaten der mechanischen Beatmung (620) umfasst: eine Aufzeichnungstabelle für Überwachungsparameter der mechanischen Beatmung (621);

die Inhalte der Unterseite für Einstellparameterdaten der mechanischen Beatmung (610) und der Unterseite für Überwachungsparameterdaten der mechanischen Beatmung (620) auf Basis mechanischer Beatmungsdaten und deren Datenkennzeichnung ermittelt werden;

die Datenseite ferner folgende optionale Unterseiten umfasst:

eine Unterseite für physiologische Parameterdaten über 24 Stunden (630), die umfasst: eine Aufzeichnungstabelle für Vitalzeichenüberwachung über 24 Stunden, Trenddiagramme für Herzfrequenz/Atemfrequenz/Blutdruck; die Inhalte der Unterseite für physiologische Parameterdaten über 24 Stunden (630) auf Basis physiologischer Parameterdaten und deren Datenkennzeichnung ermittelt werden;

eine Unterseite für Blutgasanalyse- und Labordiagnosedaten über 24 Stunden (640) umfasst: eine Aufzeichnungstabelle für Blutgasanalyseüberwachung über 24 Stunden, Trenddiagramme für Sauerstoffpartialdruck/Kohlendioxidpartialdruck/Bicarbonat sowie eine Datentabelle für Labordiagnosen; die Inhalte der Unterseite für Blutgasanalyse- und Labordiagnosedaten über 24 Stunden (640) auf Basis Blutgasanalysedaten, Labordiagnosedaten und deren Datenkennzeichnung ermittelt werden;

eine Unterseite für Alarmereignisdaten (650) umfasst: eine Aufzeichnungstabelle für Alarmereignisse, eine Verteilungskarte für Alarmereignisse über 24 Stunden, eine Tabelle für umfassende Analyse von Alarmereignissen;

die Unterseite für Alarmereignisdaten (650) auf Basis der in den Daten zur mechanischen Beatmungstherapie gekennzeichneten abnormalen Ereignisse ermittelt wird;

eine erweiterte Unterseite für Bilddaten (660), die zur Anzeige der Bilddaten dient.

7. Das Verfahren zur Datenverwaltung in der mechanischen Beatmungstherapie nach Anspruch 6, wobei die Analyseseite ferner eine Grafikseite umfasst; die Grafikseite unterteilt in einen Bereich für mechanische Beatmungswellenformdaten (701), einen Bereich für mechanische Beatmungsschleifendiagramme (703) und einen Bereich für Wellenformdaten physiologischer Parameter (702) ist;

der Bereich für mechanische Beatmungswellenformdaten (701) kontinuierliche Wellenformdiagramme normaler mechanischer Beatmungsdaten und Wellenformdiagramme abnormaler mechanischer Beatmungsereignisse umfasst;

der Bereich für mechanische Beatmungsschleifendiagramme (703) Druck-Volumen-Schleifendiagramme und Fluss-Volumen-Schleifendiagramme umfasst;

die Inhalte des Bereichs für mechanische Beatmungswellenformdaten (701) und des Bereichs für mechanische Beatmungsschleifendiagramme (703) auf Basis mechanischer Beatmungsdaten und deren Datenkennzeichnung ermittelt werden;

der Bereich für Wellenformdaten physiologischer Parameter (702) kontinuierliche Wellenformdiagramme physiologischer Parameterdaten umfasst, die mit den Wellenformdiagrammen der mechanischen Beatmungsdaten synchronisiert sind;

die Inhalte des Bereichs für Wellenformdaten physiologischer Parameter (702) auf Basis physiologischer Parameterdaten und deren Datenkennzeichnung ermittelt werden.

8. Das Verfahren zur Datenverwaltung in der mechanischen Beatmungstherapie nach einem der Ansprüche 5-7, wobei der Befehl zur Berichtserstellung ein benutzerdefinierter Berichtserstellungsbefehl ist;

der benutzerdefinierte Berichtserstellungsbefehl einen oder mehrere benutzerdefinierte Parameter enthält, zur Erstellung der Übersichtsseite (400) und der angepassten Analyseseite;

die benutzerdefinierten Parameter die Trenddiagrammseite (500), die Datenseite oder die Grafikseite umfassen.

9. Das Verfahren zur Datenverwaltung in der mechanischen Beatmungstherapie nach einem der Ansprüche 5-7, wobei das Verfahren ferner umfasst:

während der Datenkennzeichnung bei Auftreten eines oder mehrerer abnormaler Ereignisse aus folgender Gruppe wird: Stillstand des Beatmungsgeräts, Erstickung, ansteigender Atemwegsdruck, Herzstillstand, Tachykardie, Bradykardie, Kammerflattern, Kammerflimmern und niedriger Blutdruck, eine Erste-Hilfe-Anweisung ausgegeben, der Informationen zu dem aufgetretenen abnormalen Ereignis enthält; oder

während der Datenkennzeichnung bei Auftreten eines oder mehrerer abnormaler Ereignisse aus folgender Gruppe wird: Patient-Beatmungsgerät-Asynchronie, hoher Atemwegsdruck, niedriger Atemwegsdruck, hoher positiver endexspiratorischer Druck, niedriger positiver endexspiratorischer Druck, hohes Minutenvolumen, niedriges Minutenvolumen, hohes Atemzugvolumen, niedriges Atemzugvolumen, hohe Atemfrequenz, niedrige Atemfrequenz, hohe Sauerstoffkonzentration, niedrige Sauerstoffkonzentration, ein Befehl zur Anpassung der Beatmungsparameter ausgegeben, der Informationen zu dem aufgetretenen abnormalen Ereignis enthält.

10. Das Verfahren zur Datenverwaltung in der mechanischen Beatmungstherapie nach einem der Ansprüche 1-4, wobei, in dem Standarddatenstrukturobjekt (201), für jeden Datentyp der mechanischen Beatmungstherapie sowie für die Patienteninformationen jeweils ein entsprechendes Datenstrukturobjekt konfiguriert ist;

die entkoppelten einzelnen Datentypen der mechanischen Beatmungstherapie sowie die Patienteninformationen jeweils in die entsprechenden Datenstrukturobjekte eingefügt werden, um standardisierte Daten zu bilden.

11. Ein System zur Datenverwaltung in der mechanischen Beatmungstherapie, wobei das System ein Anwendungssoftwaresystem (801, 1204), klinische Datenterminals (802, 901, 1206) und einen Datenbankserver (803, 1203) umfasst;

wobei das Anwendungssoftwaresystem (801, 1204) umfasst:

ein Modul zur Datenerfassung und Standardisierung, das dazu dient, Patienteninformationen und in Echtzeit empfangene Daten zur mechanischen Beatmungstherapie jeweils in ein Standarddatenstrukturobjekt (201) einzufügen, um standardisierte Daten zu bilden; die Daten zur mechanischen Beatmungstherapie Beatmungsdaten umfassen; wobei die Daten zur mechanischen Beatmungstherapie ferner einen oder mehrere der folgenden Datentypen umfassen: Daten physiologischer Parameter, Blutgasanalysedaten, Labordiagnosedaten, Bildgebungsdaten, klinische Diagnose- und Behandlungsinformationen sowie die Geräte-ID zur Ausgabe der Daten zur mechanischen Beatmungstherapie; wobei die Daten physiologischer Parameter von Mehrparameter-Monitoren (808, 1208) ausgegeben werden; Patienteninformationen, Blutgasanalysedaten, Labordiagnosedaten, Bildgebungsdaten und klinische Diagnose- und Behandlungsinformationen jeweils von einem klinischen Informationssystem (810, 1209) ausgegeben werden; die mechanischen Beatmungsdaten von Beatmungsgeräten (807) ausgegeben werden;

ein Datenbereinigungsmodul, das dazu dient, eine Datenbereinigung der standardisierten Daten durchzuführen, um einen medizinischen Datensatz zu erhalten;

ein Datenkennzeichnungsmodul, das dazu dient, eine Datenkennzeichnung für die Daten zur mechanischen Beatmungstherapie in dem medizinischen Datensatz durchzuführen;

die Datenkennzeichnung umfasst: Markieren der Amplituden, Frequenzen und Formen von Wellenformdaten, Markieren der Messwerte numerischer Daten und Markieren abnormaler Ereignisse;

ein Berichtserstellungsmodul, das dazu dient, bei Empfang eines Befehls zur Berichtserstellung auf Basis des medizinischen Datensatzes mit Datenkennzeichnung einen Analysebericht für Daten zur mechanischen Beatmungstherapie zu generieren;

klinische Datenterminals (802, 901, 1206), die dazu dienen, den Befehl zur Berichtserstellung auszugeben und ferner dazu dienen, den Analysebericht für Daten zur mechanischen Beatmungstherapie zu empfangen und anzuzeigen;

einen Datenbankserver (803, 1203), der dazu dient, die standardisierten Daten, den medizinischen Datensatz und den Analysebericht für Daten zur mechanischen Beatmungstherapie bereichsweise zu speichern;

wobei in den mechanischen Beatmungsdaten die Wellenformdaten umfassen: mechanische Beatmungsdruck-Wellenformdaten, Volumenwellenformdaten, Flusswellenformdaten und endtidale Kohlendioxidwellenformdaten; numerische Daten umfassen: Atemwegswiderstand, Atemarbeit, Compliance, Beatmungsparameter und Atemfrequenz;

bei der Datenkennzeichnung der mechanischen Beatmungsdaten,

die markierten Formen der Wellenformdaten umfassen: Beginn der Inspirationsphase, Inspirationsphase, Ende der Inspirationsphase, Beginn der Exspirationsphase, Exspirationsphase, Ende der Exspirationsphase, Atemwegsspitzendruck, exspiratorischer Spitzenfluss jeder mechanischen Beatmungswelle sowie Inspirationsbasislinie, Höhe des Exspirationsplateaus, Beginn und Ende des Exspirationsplateaus der endtidalen Kohlendioxidwelle;

die markierten abnormalen Ereignisse umfassen: Patient-Beatmungsgerät-Asynchronie, hoher/niedriger Atemwegsdruck, hoher/niedriger PEEP, hohes/niedriges Minutenvolumen, hohe/niedrige Sauerstoffkonzentration, hohe/niedrige Atemfrequenz, hoher/niedriger endtidaler Kohlendioxidpartialdruck, hohes/niedriges Atemzugvolumen, Ausfall des Beatmungsgeräts, Erstickung und ansteigender Atemwegsdruck;
wobei das System dazu dient, Patient-Beatmungsgerät-Asynchronie auf Basis eines neuronalen Netzwerkmodells zu erkennen, indem folgende Schritte ausgeführt werden:

Aufbauen der Modelleingabe $T = \langle S_1, \cdots, S_c, \cdots, S_C \rangle$ auf Basis der Druck-Zeit-Kurve der mechanischen Beatmungsdruck-Wellenformdaten, der Volumen-Zeit-Kurve der mechanischen Beatmungsvolumen-Wellenformdaten und der Fluss-Zeit-Kurve der mechanischen Beatmungsfluss-Wellenformdaten; wobei $S_c$ der Eingabevektor des c-ten Abtastpunkts ist, C die Gesamtanzahl der Abtastpunkte darstellt, die das neuronale Netzwerkmodell einmal verarbeitet, d. h. die Modelleingabelänge;

$$S_C = (S_C^1, S_C^2, S_C^3), \quad S_C^1, S_C^2, S_C^3$$ , jeweils die Werte für Fluss, Druck und Volumen des c-ten Abtastpunkts darstellen;

Durchführen von zwei Gleitfensterverarbeitungen zur Merkmalsanreicherung, um eine zeitliche Reihe nach Gleitfensterverarbeitung zu erhalten; wobei,

bei der ersten Gleitfensterverarbeitung, Ausführung: Durchführen einer Gleitfensterverarbeitung der Modelleingabe T mit der Schrittweite b/2, wobei b die erste Gleitfensterbreite darstellt; nach der ersten Gleitfensterverarbeitung sich die Modelleingabelänge von C auf C' ändert, der Wertebereich von c [1, C'] wird;

$$C' = \frac{2*C - b}{b};$$

nach der ersten Gleitfensterverarbeitung auf Basis der *l*-ten Wellenformdaten aller Abtastpunkte im c-ten Gleitfenster die euklidische Distanz der *l*-ten Wellenformdaten im euklidischen Matrixvektor $NOR_C^l$

berechnet wird, d. h. $$NOR_C^l = \sqrt{\left(S_c^l\right)^2 + \left(S_{c+1}^l\right)^2 + \cdots + \left(S_{c+b-1}^l\right)^2}$$ ; *l* jeweils 1, 2, 3 annimmt; schließlich der euklidische Matrixvektor $NOR_C = (NOR_C^1, NOR_C^2, NOR_C^3)$ erhalten wird; auf Basis der c-ten Wellenformdaten aller Abtastpunkte im c-ten Gleitfenster wird der Distanzunterschied der *l*-ten Wellenformdaten im Distanzdifferenzvektor $DON_C^l$ berechnet, d. h.

$$DON_C^l = NOR_{c+1}^l - NOR_c^l \quad ; \quad$$ schließlich der Distanzdifferenzvektor als

$$DON_C = (DON_C^1, DON_C^2, DON_C^3)$$ dargestellt wird; daher die zeitliche Reihe nach der ersten Gleitfensterverarbeitung $T' = \langle G_1, ..., G_c, ..., G_C \rangle$ ist, wobei $G_c = \langle NOR_c, DON_c \rangle$;

bei der zweiten Gleitfensterverarbeitung, Ausführung: Durchführen einer erneuten Gleitfensterverarbeitung der zeitlichen Reihe *T'* mit der Schrittweite *f/2*, wobei *f* die zweite Gleitfensterbreite darstellt; nach der zweiten Gleitfensterverarbeitung sich die Modelleingabelänge von *C'* auf C " ändert, der Wertebereich von c [1, *C'*]

$$C'' = \frac{2 * C' - f}{f}$$ wird; ; während der zweiten Gleitfensterverarbeitung abgeleitete Merkmale aller euklidischen Matrixvektoren und Distanzdifferenzvektoren im zweiten Gleitfenster ermittelt werden, um die zeitliche Reihe nach der zweiten Gleitfensterverarbeitung zu erhalten;

Eingeben der zeitlichen Reihe nach Gleitfensterverarbeitung in das neuronale Netzwerkmodell; das neuronale Netzwerkmodell nach Verarbeitung normale Ereignisse und abnormale Beatmungsgeräteereignisse ausgibt, einschließlich Art und Zeitpunkt der Patient-Beatmungsgerät-Asynchronie.

12. Das System zur Datenverwaltung in der mechanischen Beatmungstherapie nach Anspruch 11, wobei das Anwendungssoftwaresystem (801, 1204) ferner ein Streaming-Verwaltungsmodul umfasst, das zur Echtzeit-Empfang von Streaming-Daten des Behandlungsorts dient; die Streaming-Daten von Bett-Seiten-Audio-Video-Geräten (809, 1205) ausgegeben werden;

der Datenbankserver (803, 1203) ferner zur bereichsweisen Speicherung der Streaming-Daten sowie zur Verknüpfung der Streaming-Daten mit dem medizinischen Datensatz und dem Analysebericht für Daten zur mechanischen Beatmungstherapie dient;

die klinischen Datenterminals (802, 901, 1206) ferner zur Echtzeit-Übertragung von Wellenformdaten, numerischen Daten, Behandlungsorten sowie des Analyseberichts für Daten zur mechanischen Beatmungstherapie aus den medizinischen Datensätzen teilweise oder aller Patienten in einem oder mehreren Krankenhausbereichen dienen.

13. Das System zur Datenverwaltung in der mechanischen Beatmungstherapie nach Anspruch 11, wobei das System ferner ein Multimode-Gateway (804, 1201) und einen Master-Steuerserver (805, 1205) umfasst; wobei:

das Multimode-Gateway (804, 1201) dazu dient, über das Netzwerk Beatmungsgeräte (807), Mehrparameter-Monitore (808, 1208), Bett-Seiten-Audio-Video-Geräte (809, 1205), klinische Informationssysteme (810, 1209) sowie klinische Datenterminals (802, 901, 1206) verschiedener Hersteller in allen Krankenhausbereichen anzubinden; das Multimode-Gateway (804, 1201) mehrere Kommunikationsprotokolle integriert, zur Realisierung der Datenkommunikation zwischen Beatmungsgeräten (807), Mehrparameter-Monitoren (808, 1208), Bett-Seiten-Audio-Video-Geräten (809, 1205), klinischen Informationssystemen (810, 1209) und dem Modul zur Datenerfassung und Standardisierung sowie zur Realisierung der Datenkommunikation zwischen dem Anwendungssoftwaresystem (801, 1204) und den klinischen Datenterminals (802, 901, 1206) am Patientenbett; der Master-Steuerserver (805, 1205) ein Multitask-Betriebssystem und das darauf laufende Anwendungssoftwaresystem (801, 1204) integriert und zur Steuerung des Betriebs des Anwendungssoftwaresystems (801, 1204) dient.

## Revendications

1. Un procédé de gestion de données thérapeutiques de ventilation mécanique, dans lequel le procédé comprend :

introduire respectivement des informations de patients et données thérapeutiques de ventilation mécanique reçues en temps réel dans un objet de structure de données standard (201), pour former données standardisées (S1) ; les données thérapeutiques de ventilation mécanique comprennent données de ventilation ; dans lesquelles les données thérapeutiques de ventilation mécanique comprennent en outre une ou plusieurs des données suivantes : données de paramètres physiologiques, données d'analyse des gaz du sang, données de diagnostic de laboratoire, données d'imagerie, informations sur le diagnostic clinique et le traitement, ainsi qu'un ID d'un appareil d'exportation des données thérapeutiques de ventilation mécanique ; dans lesquelles les données de paramètres physiologiques sont sorties par moniteurs multiparamétriques (808, 1208) ; les informations de patients, les données d'analyse des gaz du sang, les données de diagnostic de laboratoire, les données d'imagerie et les informations sur le diagnostic clinique et le traitement sont toutes sorties par un système d'information clinique (810, 1209) ; les données de ventilation sont sorties par des appareils de ventilation (807) ;
effectuer un nettoyage des données sur les données standardisées pour obtenir un ensemble de données médicales (S2) ;
effectuer une annotation des données sur les données thérapeutiques de ventilation mécanique dans l'ensemble de données médicales (S3) ; l'annotation des données comprend : l'annotation des amplitudes, des fréquences et des formes des données ondulatoires, l'annotation des valeurs quantitatives des données numériques, et l'annotation des événements anormaux ;
lorsqu'une instruction de génération de rapport est reçue, générer un rapport d'analyse des données thérapeutiques de ventilation mécanique à partir de l'ensemble de données médicales avec l'étiquetage des données (S4) ;
dans lequel dans les données de ventilation mécanique, les données ondulatoires comprennent : données ondulatoires de pression de ventilation mécanique, données ondulatoires de volume, données ondulatoires de débit et données ondulatoires de dioxyde de carbone en fin d'expiration ; les données numériques comprennent : résistance de voie respiratoire, travail respiratoire, compliance, paramètres de ventilation et fréquence respiratoire ;
au cours de l'annotation des données sur les données de ventilation mécanique,
les formes des données ondulatoires annotées comprennent : début de phase inspiratoire, phase inspiratoire, fin de phase inspiratoire, début de phase expiratoire, phase expiratoire, fin de phase expiratoire, pression de crête de voie respiratoire, débit de crête expiratoire de chaque onde de ventilation mécanique, ainsi que ligne de base inspiratoire, hauteur de plateau expiratoire, début et fin de plateau expiratoire de forme d'onde de dioxyde de carbone en fin d'expiration ;

les événements anormaux annotés comprennent: désynchronisation patient-ventilateur, hausse/baisse de pression de voie respiratoire, hausse/baisse de pression positive en fin d'expiration, hausse/baisse de ventilation minute, hausse/baisse de concentration en oxygène, hausse/baisse de fréquence respiratoire, hausse/baisse de pression partielle en dioxyde de carbone en fin d'expiration, hausse/baisse de volume courant, arrêt d'un ventilateur, étouffement et augmentation de pression de voie respiratoire ;

dans lequel le procédé détermine la désynchronisation patient-ventilateur sur la base d'un modèle de réseau neuronal, en exécutant les étapes suivantes :

construire des entrées de modèle $T = \langle S_1, \cdots, S_c, \cdots, S_C \rangle$ sur la base d'une courbe pression-temps des données ondulatoires de pression de ventilation mécanique, d'une courbe volume-temps des données ondulatoires de volume de ventilation mécanique, et d'une courbe débit-temps des données ondulatoires de débit de ventilation mécanique,; dans laquelle $S_c$ représente un vecteur d'entrée du $C$-ème point d'échantillonnage, $C$ représente un nombre total de points d'échantillonnage traités en une seule fois par le modèle de réseau neuronal, c'est-à-dire une longueur d'entrée de modèle ;

$$S_c = \left(S_c^1, S_c^2, S_c^3\right)$$ , $S_c^1$、 $S_c^2$、 $S_c^3$ représentent respectivement des valeurs de débit, de pression et de volume du $C$-ème point d'échantillonnage ;

effectuer deux fois un traitement par fenêtre glissante en enrichissant des caractéristiques, pour obtenir une série temporelle après chaque traitement par fenêtre glissante, dans lequel :

au cours du premier traitement par fenêtre glissante, exécuter : effectuer un traitement par fenêtre glissante sur l'entrée de modèle $T$, en utilisant $b/2$ comme pas d'une fenêtre glissante, où $b$ représente une largeur du premier traitement par fenêtre glissante ; après le premier traitement par fenêtre glissante, la longueur d'entrée de modèle passe de $C$ à $C'$, et la plage de valeurs de $C$ devient [1, $C'$];

$$C' = \frac{2 * C - b}{b}$$ ;

après le premier traitement par fenêtre glissante, sur la base des données ondulatoires de la $l$-ième classe parmi tous les points d'échantillonnage dans la $C$-ème fenêtre glissante, calculer une distance euclidienne $NOR_c^l$ des données ondulatoires de la $l$-ième classe dans un vecteur de matrice euclidienne, c'est-à-dire, $$NOR_c^l = \sqrt{\left(S_c^l\right)^2 + \left(S_{c+1}^l\right)^2 + \cdots + \left(S_{c+b-1}^l\right)^2}$$ , une valeur de $l$ respectivement est 1, 2, 3 ; obtenir finalement le vecteur matrice euclidien $$NOR_c = \left(NOR_c^1, NOR_c^2, NOR_c^3\right)$$ ; sur la base des données ondulatoires de la $l$-ième classe parmi tous les points d'échantillonnage dans la $C$-ème fenêtre glissante, calculer une différence de distance $DON_c^l$ des données ondulatoires de la $l$-ième classe dans un vecteur de différence de distance, c'est-à-dire, $$DON_c^l = NOR_{c+1}^l - NOR_c^l$$ , le vecteur de différence de distance obtenu finalement est représenté par $$DON_c = \left(DON_c^1, DON_c^2, DON_c^3\right)$$ ; par conséquent, la série temporelle après le premier traitement par fenêtre glissante est $T' = \langle G_1, \cdots, G_c, \cdots, G_{C'} \rangle$, où $G_c = \langle NOR_c, DON_c \rangle$;

au cours du deuxième traitement par fenêtre glissante, exécuter : effectuer encore un traitement par fenêtre glissante sur la série temporelle $T'$, en utilisant $f/2$ comme pas d'une fenêtre glissante, où $f$ représente une largeur du deuxième traitement par fenêtre glissante ; après le deuxième traitement par fenêtre glissante, la longueur d'entrée de modèle passe de $C'$ à $C''$, et une plage de valeurs de $C$ devient [1, $C''$]; $$C'' = \frac{2 * C' - f}{f}$$ ; au cours du deuxième traitement par fenêtre glissante, obtenir des caractéristiques dérivées de tous les vecteurs de la matrice euclidienne et les vecteurs de différence de distance dans la deuxième fenêtre glissante, afin d'obtenir la série temporelle après le deuxième traitement par fenêtre glissante ;

entrer la série temporelle après les traitements par fenêtre glissante dans le modèle de réseau neuronal, après les traitements, le modèle de réseau neuronal génère des événements normaux ainsi que des événements anormaux d'un ventilateur, comprenant un type et un moment d'occurrence de la désynchronisation patient-ventilateur.

2. Le procédé de gestion de données thérapeutiques de ventilation mécanique, selon la revendication 1, dans lequel dans les données de paramètres physiologiques, les données ondulatoires comprennent : données ondulatoires électrocardiographiques, données ondulatoires respiratoires, données ondulatoires de photopléthysmographie et données ondulatoires de pression artérielle invasive ; les données numériques comprennent : fréquence cardiaque, pression artérielle, saturation en oxygène du sang et température corporelle ;

au cours de l'annotation des données sur les données de paramètres physiologiques,
les formes des données ondulatoires électrocardiographiques annotées comprennent : onde P, groupe d'ondes QRS, onde T, segment ST, intervalle PR, intervalle RR et intervalle QT de chaque forme d'onde périodique électrocardiographique ;
les formes des données ondulatoires respiratoires annotées comprennent : crêtes et creux de chaque forme d'onde périodique respiratoire ;
les formes des données ondulatoires de photopléthysmographie annotées comprennent : crêtes et creux de chaque forme d'onde périodique de pouls ;
les formes des données ondulatoires de pression artérielle invasive annotées comprennent : crêtes et creux de chaque forme d'onde périodique de contraction-dilatation artérielle ;
les événements anormaux annotés comprennent : tachycardie, bradycardie, arrêt cardiaque, flutter auriculaire, fibrillation auriculaire, flutter ventriculaire, fibrillation ventriculaire, bloc de conduction auriculo-ventriculaire, élévation/dépression du segment ST, hypertension/hypotension, hyperthermie/hypothermie et baisse de saturation en oxygène.

3. Le procédé de gestion de données thérapeutiques de ventilation mécanique, selon la revendication 2, dans lequel dans les données d'analyse des gaz du sang, les données numériques comprennent : pression partielle en oxygène, pression partielle en dioxyde de carbone, quantité totale de dioxyde de carbone, concentration en ions potassium, concentration en ions sodium, concentration en ions bicarbonate et pH ;

au cours de l'annotation des données sur les données d'analyse des gaz du sang,
les événements anormaux annotés comprennent : hausse/baisse de pression partielle d'oxygène, hausse/baisse de pression partielle en dioxyde de carbone, hausse/baisse de quantité totale de dioxyde de carbone, hausse/baisse de concentration en ions potassium, hausse/baisse de concentration en ions sodium et hausse/baisse de pH.

4. Le procédé de gestion de données thérapeutiques de ventilation mécanique, selon la revendication 3, dans lequel dans les données de diagnostic de laboratoire, les données numériques comprennent : dénombrement de leucocytes, dénombrement de globules rouges, dénombrement de plaquettes, hémoglobine, hématocrite, glycémie, taux de prothrombine et taux de prothrombine partielle activée ;

au cours de l'annotation des données sur les données de diagnostic de laboratoire,
les événements anormaux annotés comprennent : hausse/baisse de dénombrement de leucocytes, hausse/baisse de dénombrement de globules rouges, hausse/baisse d'hémoglobine, hausse/baisse d'hématocrite, hausse/baisse de glycémie, hausse/baisse de dénombrement de plaquettes, hausse/baisse de taux de prothrombine et hausse/baisse de taux de prothrombine partielle activée.

5. Le procédé de gestion de données thérapeutiques de ventilation mécanique, selon la revendication 4, dans lequel un cadre du rapport d'analyse des données thérapeutiques de ventilation mécanique comprend une page de synthèse du rapport (400) et une page d'analyse du rapport ; dans lequel la page de synthèse du rapport (400) est divisée en une zone d'informations de base (401), une zone de données de ventilation mécanique (402) et une zone de conclusion d'analyse (403) ;

la zone d'informations de base (401) comprend au moins des informations de patients et des informations de points temporels clés ; et comprend en outre les options suivantes : les informations sur le diagnostic clinique, l'ID de l'appareil d'exportation des données thérapeutiques de ventilation mécanique, ainsi que des informations de modes et configurations de ventilation mécanique ;

la zone de données de ventilation mécanique (402) comprend au moins des résultats de l'analyse respiratoire, résultats d'analyse des événements de désynchronisation patient-ventilateur, résultats d'analyse de variations de résistance de voie respiratoire, résultats d'analyse de variations de compliance et résultats d'analyse d'alertes de lésion pulmonaire ; chaque résultat d'analyse de la zone de données de ventilation mécanique (402) est obtenu sur la base des données de ventilation mécanique et de leur annotation des données ; les résultats de l'analyse respiratoire comprennent des indices de respiration rapide et superficielle ;

la zone de conclusion d'analyse (403) sert à présenter une description et une conclusion de l'analyse des données thérapeutiques de ventilation mécanique ;

la page d'analyse du rapport sert à présenter sous forme de graphique un ou plusieurs rapports d'analyse détaillés des données thérapeutiques de ventilation mécanique.

6. Le procédé de gestion de données thérapeutiques de ventilation mécanique, selon la revendication 5, dans lequel la page d'analyse du rapport comprend une page de graphique de tendance (500) et une page de données ;
la page de graphique de tendance (500) comprend :

zone d'analyse de tendance pour la pression, le volume et le débit ventilatoire des données de ventilation mécanique ;
une zone d'analyse de tendance pour la fréquence respiratoire (504) ;
une zone d'analyse de tendance pour la concentration en oxygène inspiré (505) ; et
une zone de description et conclusion d'analyse de tendance (506) servant à synthétiser la direction de chaque zone d'analyse des tendances, afin de fournir des descriptions d'analyse des tendances et les conclusions correspondantes ;
les contenus de chaque zone d'analyse de tendance sur la page de graphique de tendance (500) sont obtenus sur la base des données de ventilation mécanique et de leur annotation des données ;
la page de données comprend au moins :

une sous-page de données de réglage de paramètres de ventilation mécanique (610) comprend : un tableau d'enregistrement de réglage de paramètres de ventilation mécanique (611), modes de ventilation de ventilation mécanique et un graphique de tendance de réglage de paramètres (612) ;
une sous-page de données de paramètres de surveillance de ventilation mécanique (620) comprend un tableau d'enregistrement de paramètres de surveillance de ventilation mécanique (621) ;
les contenus de la sous-page de données de réglage de paramètres de ventilation mécanique (610) et de la sous-page de données de paramètres de surveillance de ventilation mécanique (620) sont obtenus sur la base des données de ventilation mécanique et de leur annotation des données ;
la page de données comprend en outre les sous-pages de données optionnelles suivantes :

une sous-page de données de paramètres physiologiques pour 24 heures (630), comprend un tableau d'enregistrement de surveillance de signes vitaux pour 24 heures et un graphique de tendance de fréquence cardiaque/rythme respiratoire/tension artérielle ; les contenus de la sous-page de données de paramètres physiologiques pour 24 heures (630) sont obtenus sur la base des données de paramètres physiologiques et de leur annotation des données ;
une sous-page de données d'analyse de gaz du sang et de diagnostic de laboratoire pour 24 heures (640) comprend un tableau d'enregistrement de surveillance des gaz du sang pour 24 heures, un graphique de tendance de pression partielle en oxygène/pression partielle en dioxyde de carbone/bicarbonate, ainsi qu'un tableau de données de diagnostic de laboratoire,
les contenus de la sous-page de données d'analyse de gaz du sang et de diagnostic de laboratoire pour 24 heures (640) sont obtenus sur la base des données d'analyse de gaz du sang, et des données de diagnostic de laboratoire et de leurs annotations de données ;
une sous-page de données d'incidents d'alarme (650), comprend un tableau d'enregistrement d'incidents d'alarme, un graphique de répartition d'incidents d'alarme pour 24 heures, et un tableau d'analyse synthétique d'incidents d'alarme ;
la sous-page de données d'incidents d'alarme (650) est obtenue sur la base des événements anormaux annotés par les données thérapeutiques de ventilation mécanique ;
une sous-page de données d'image étendue (660), servant à présenter les données d'imagerie.

7. Le procédé de gestion de données thérapeutiques de ventilation mécanique, selon la revendication 6, dans lequel la page d'analyse du rapport comprend en outre une page graphique ; la page graphique est divisée en zones de données ondulatoires de ventilation mécanique (701), une zone de diagramme de boucle de ventilation mécanique (703) et une zone de données ondulatoires de paramètres physiologiques (702) ; dans lesquelles

la zone de données ondulatoires de ventilation mécanique (701) comprend graphiques de forme d'onde de données de ventilation mécanique normaux continues et graphiques de forme d'onde de données de ventilation mécanique anormaux ;
la zone de diagramme de boucle de ventilation mécanique (703) comprend un diagramme pression-volume et un diagramme débit-volume tracés en combinaison des données de pression, du volume et du débit de ventilation mécanique ;
les contenus de la zone de données ondulatoires de ventilation mécanique (701) et de la zone de diagramme de boucle de ventilation mécanique (703) sont obtenus sur la base des données de ventilation mécanique et de leur annotation des données ;
la zone de données ondulatoires de paramètres physiologiques (702) comprend graphiques de données de paramètres physiologiques continus synchronisés avec les graphiques de forme d'onde de données de ventilation mécanique ;
le contenu de la zone de données ondulatoires de paramètres physiologiques (702) est obtenu sur la base des données de paramètres physiologiques et de leur annotation des données.

8. Le procédé de gestion de données thérapeutiques de ventilation mécanique, selon l'une quelconque des revendications 5 à 7, dans lequel l'instruction de génération de rapport est une instruction de génération de rapport personnalisée ;

l'instruction de génération de rapport personnalisé comprend un ou plusieurs paramètres personnalisés, servant à générer une page de résumé de rapport (400), ainsi qu'une page d'analyse de rapport concordant avec le paramètre personnalisé ;
les paramètres personnalisés comprennent la page de graphique de tendance (500), la page de données ou la page graphique.

9. Le procédé de gestion de données thérapeutiques de ventilation mécanique, selon l'une quelconque des revendications 5 à 7, dans lequel le procédé comprend en outre :

au cours de l'annotation des données, une instruction de secours est envoyée lorsqu'un ou plusieurs événements anormaux tels qu'arrêt de ventilateur, étouffement, augmentation de pression d'une voie respiratoire, arrêt cardiaque, tachycardie, bradycardie, flutter ventriculaire, fibrillation ventriculaire et hypotension survenant, l'instruction de secours contient les informations des événements anormaux survenus ; ou
au cours de l'annotation des données, une instruction d'ajustement des paramètres du ventilateur est envoyée lorsqu'un ou plusieurs événements anormaux tels que désynchronisation patient-ventilateur, hausse de pression de voie respiratoire ; baisse de pression de voie respiratoire, hausse de pression positive en fin d'expiration, baisse de pression positive en fin d'expiration, hausse de ventilation minute, baisse de ventilation minute, hausse de volume courant, baisse de volume courant, hausse de fréquence respiratoire, baisse de fréquence respiratoire, hausse de concentration en oxygène et baisse de concentration en oxygène survenant, dans lequel l'instruction d'ajustement des paramètres du ventilateur contient les informations des événements anormaux survenus.

10. Le procédé de gestion de données thérapeutiques de ventilation mécanique, selon l'une quelconque des revendications 1 à 4, dans lequel, dans l'objet de structure de données standard (201), un objet de structure de données correspondant est configuré à chaque donnée thérapeutique de ventilation mécanique et à chaque information de patients respectivement ;
chaque donnée thérapeutique de ventilation mécanique et chaque information de patients découplées sont introduites dans l'objet de structure de données correspondant, afin de former données standardisées.

11. Un système de gestion de données thérapeutiques de ventilation mécanique, dans lequel le système comprend un système logiciel applicatif (801, 1204), terminaux de données cliniques (802, 901, 1206) et un serveur de base de données (803, 1203) ; dans lesquels,
le système logiciel applicatif (801, 1204) comprend :

un module de collecte et de normalisation des données, servant à introduire respectivement les informations de patients et les données thérapeutiques de ventilation mécanique reçues en temps réel dans l'objet de structure de données standard (201), pour former données standardisées; les données thérapeutiques de ventilation mécanique comprennent données de ventilation ; dans lequel les données thérapeutiques de ventilation mécanique comprennent en outre une ou plusieurs des données suivantes : données de paramètres physiologiques, données d'analyse des gaz du sang, données de diagnostic de laboratoire, données d'imagerie, informations sur le diagnostic clinique et le traitement, ainsi que l'ID de l'appareil d'exportation des données thérapeutiques de ventilation mécanique : dans lesquelles les données de paramètres physiologiques sont sorties par moniteurs multiparamétriques (808, 1208) ; les informations de patients, les données d'analyse des gaz du sang, les données de diagnostic de laboratoire, les données d'imagerie et les informations sur le diagnostic clinique et le traitement sont toutes sorties par un système d'information clinique (810, 1209) ; les données de ventilation sont sorties par des appareils de ventilation (807);

un module de nettoyage des données servant à effectuer nettoyage des données sur les données standardisées pour obtenir un ensemble de données médicales ;

un module d'annotation des données servant à effectuer annotation des données sur les données thérapeutiques de ventilation mécanique dans l'ensemble de données médicales ;

l'annotation des données comprend : annotation des amplitudes, des fréquences et des formes des données ondulatoires, annotation des valeurs quantitatives des données numériques, et annotation des événements anormaux ;

un module de génération de rapport servant à au moment de recevoir une instruction de génération de rapport, générer un rapport d'analyse des données thérapeutiques de ventilation mécanique sur la base de l'ensemble de données médicales avec l'annotation des données ;

les terminaux de données cliniques (802, 901, 1206) servant à envoyer l'instruction de génération de rapport, et servant en outre à recevoir et représenter le rapport d'analyse des données thérapeutiques de ventilation mécanique ;

le serveur de base de données (803, 1203) servant à stocker en partition les données standardisées, l'ensemble de données médicales et le rapport d'analyse des données thérapeutiques de ventilation mécanique ;

dans lequel dans les données de ventilation mécanique, les données ondulatoires comprennent : données ondulatoires de pression de ventilation mécanique, données ondulatoires de volume de ventilation mécanique, données ondulatoires de débit de ventilation mécanique et données ondulatoires de dioxyde de carbone en fin d'expiration ; les données numériques comprennent : résistance de voie respiratoire, travail respiratoire, compliance, paramètres de ventilation et fréquence respiratoire ;

au cours de l'annotation des données sur les données de ventilation mécanique,

les formes des données ondulatoires annotées comprennent : début de phase inspiratoire, phase inspiratoire, fin de phase inspiratoire, début de phase expiratoire, phase expiratoire, fin de phase expiratoire, pression de crête de voie respiratoire, débit de crête expiratoire de chaque onde de ventilation mécanique, ainsi que ligne de base inspiratoire, hauteur de plateau expiratoire, début et fin de plateau expiratoire de la forme d'onde de dioxyde de carbone en fin d'expiration ;

les événements anormaux annotés comprennent: désynchronisation patient-ventilateur, hausse/baisse de pression de voie respiratoire, hausse/baisse de pression positive en fin d'expiration, hausse/baisse de ventilation minute, hausse/baisse de concentration en oxygène, hausse/baisse de fréquence respiratoire, hausse/baisse de pression partielle en dioxyde de carbone en fin d'expiration, hausse/baisse de volume courant, arrêt de ventilateur, étouffement et augmentation de pression de voie respiratoire ;

dans lequel le système servant pour déterminer la désynchronisation patient-ventilateur sur la base d'un modèle de réseau neuronal, en exécutant les étapes de :

sur la base d'une courbe pression-temps des données ondulatoires de pression de ventilation mécanique, d'une courbe volume-temps des données ondulatoires de volume de ventilation mécanique, et d'une courbe débit-temps des données ondulatoires de débit de ventilation mécanique, construire des entrées de modèle $T = \langle S_1, \cdots, S_c, \cdots, S_C \rangle$, dans lesquelles $S_c$ représente un vecteur d'entrée du $C$ - ème point d'échantillonnage, $C$ représente un nombre total de points d'échantillonnage traités en une seule fois par le modèle de réseau neuronal, c'est-à-dire une longueur d'entrée de modèle ; $S_c = \left( S_c^1, S_c^2, S_c^3 \right)$, $S_c^1$、 $S_c^2$、 $S_c^3$ représentent respectivement des valeurs de débit, de pression et de volume du $C$-ème point d'échantillonnage ;

effectuer deux fois un traitement par fenêtre glissante en enrichissant des caractéristiques, pour obtenir une série temporelle après chaque traitement par fenêtre glissante, dans lequel :

au cours du premier traitement par fenêtre glissante : effectuer un traitement par fenêtre glissante sur l'entrée de modèle $T$, en utilisant $b/2$ comme pas d'une fenêtre glissante, où $b$ représente une largeur du premier traitement par fenêtre glissante ; après le premier traitement par fenêtre glissante, la longueur d'entrée de modèle passe de *C* à *C'*, et une plage de valeurs de *C* devient [1, *C'*]; $C' = \dfrac{2*C-b}{b}$ ;

après le premier traitement par fenêtre glissante, sur la base des données ondulatoires de la *l*-ième classe parmi tous les points d'échantillonnage dans la *C* - ème fenêtre glissante, calculer une distance euclidienne $NOR_c^l$ des données ondulatoires de la *l*-ième classe dans un vecteur de matrice euclidienne, c'est-à-dire, $NOR_c^l = \sqrt{\left(S_c^l\right)^2 + \left(S_{c+1}^l\right)^2 + \cdots + \left(S_{c+b-1}^l\right)^2}$ , une valeur de *l* respectivement est 1, 2, 3 ; obtenir finalement le vecteur matrice euclidien

$NOR_c = \left(NOR_c^{\,1}, NOR_c^{\,2}, NOR_c^{\,3}\right)$ ; sur la base des données ondulatoires de la *l*-ième classe parmi tous les points d'échantillonnage dans la *C* -ème fenêtre glissante, on calcule une différence de distance $DON_c^{\,l}$ des données ondulatoires de la *l*-ième classe dans un vecteur de différence de distance, c'est-à-dire, $DON_c^{\,l} = NOR_{c+1}^{\,l} - NOR_c^{\,l}$ ; le vecteur de différence de distance obtenu finalement est représenté par $DON_c = \left(DON_c^{\,1}, DON_c^{\,2}, DON_c^{\,3}\right)$ ;

par conséquent, la série temporelle après le premier traitement par fenêtre glissante est *T'* = $\langle G_1, \cdots, G_c, \cdots, G_c\rangle$, où $G_c = \langle NOR_c, DON_c\rangle$;

au cours du deuxième traitement par fenêtre glissante, exécuter : effectuer encore un traitement par fenêtre glissante sur la série temporelle *T'*, en utilisant *f* / 2 comme pas d'une fenêtre glissante, où *f* représente une largeur du deuxième traitement par fenêtre glissante ; après le deuxième traitement par fenêtre glissante, la longueur d'entrée de modèle passe de *C'* à *C"* , et une plage de valeurs de *C* devient

[1, *C"*]; $C'' = \dfrac{2*C'-f}{f}$ ; au cours du deuxième traitement par fenêtre glissante, obtenir des caractéristiques dérivées de tous les vecteurs de la matrice euclidienne et les vecteurs de différence de distance dans la deuxième fenêtre glissante, afin d'obtenir la série temporelle après le deuxième traitement par fenêtre glissante ; et

entrer la série temporelle après les deux traitements par fenêtre glissante dans le modèle de réseau neuronal, la série temporelle ensuite entre événements normaux ainsi qu'événements anormaux d'un ventilateur, comprenant un type et un moment d'occurrence de la désynchronisation patient-ventilateur.

12. Le système de gestion de données thérapeutiques de ventilation mécanique, selon la revendication 11, dans lequel le système logiciel applicatif (801, 1204) comprend en outre un module de gestion de streaming, le module de gestion de streaming sert à recevoir en temps réel des données de streaming de scénarios ; les données de streaming sont sorties par un équipement audio et vidéo au chevet (809, 1205) ;

le serveur de base de données (803, 1203) sert en outre à stocker en partition les données de streaming et à associer les données de streaming avec l'ensemble de données médicales et le rapport d'analyse des données thérapeutiques de ventilation mécanique ;
les terminaux de données cliniques (802, 901, 1206) servant en outre à effectuer un streaming en direct sur les données ondulatoires, les données numériques, et les scénarios de traitement dans les données thérapeutiques de ventilation mécanique de l'ensemble de données médicales ainsi que les rapports d'analyse des données thérapeutiques de ventilation mécanique pour certains ou tous les patients dans une ou plusieurs unités de soins, en temps réel.

13. Le système de gestion de données thérapeutiques de ventilation mécanique, selon la revendication 11, dans lequel le système comprend en outre une passerelle multimodale (804, 1201) et un serveur de contrôle principal (805,1205) ; dans lequel

la passerelle multimodale (804, 1201) sert à connecter des appareils de ventilation (807), moniteurs multiparamétriques (808, 1208), équipement audio-vidéo au chevet (809, 1205), systèmes d'information clinique (810, 1209) et les terminaux de données cliniques (802, 901, 1206) par différents fabricants, dans des différents unités de soins d'un hôpital ; la passerelle multimodale (804,1201) intègre plusieurs protocoles de communication pour réaliser une communication de données entre les appareils de ventilation (807), les moniteurs multiparamétriques (808,1208), l'équipement audio-vidéo au chevet (809, 1205), les systèmes d'information clinique (810, 1209) et le module de collecte et de normalisation des données, ainsi que à réaliser une communication de données entre le système logiciel applicatif (801,1204) et les terminaux de données cliniques (802, 901, 1206) où se trouvent les patients ;

le serveur de contrôle principal (805,1205), incorporant un système d'exploitation multitâche et un système logiciel applicatif (801, 1204) fonctionnant sur le système d'exploitation multitâche, sert à contrôler le fonctionnement du système logiciel applicatif (801, 1204).

S1

Putting patient information and real-time received mechanical ventilation therapy data into a standard data structure object respectively to form standardized data; the mechanical ventilation therapy data comprises mechanical ventilator data

S2

Performing data cleaning on the standardized data to obtain a medical data set

S3

Performing data labeling on the mechanical ventilation therapy data in the medical data set; the data labeling comprises: marking amplitudes, frequencies, and shapes of waveform data, marking measurement values of numerical data, and marking abnormal events

S4

When a report generation instruction is received, generating a mechanical ventilation therapy data analysis report based on the medical data set with the data labeling

S5

During the data labeling process, a first aid instruction is issued when one or more abnormal events of ventilators stop working, asphyxia, increasing airway pressure, cardiac arrest, tachycardia, bradycardia, ventricular flutter, ventricular fibrillation and hypotension occur, the first aid instruction carries the information about the abnormal events that occur

S6

During the data labeling process, a ventilator parameters adjustment instruction is issued when one or more abnormal events of patient-ventilator asynchronous event, high airway pressure, low airway pressure, high positive end expiratory pressure, low positive end expiratory pressure, high minute ventilation, low minute ventilation, high tidal volume, low tidal volume, high respiratory rate, low respiratory rate, high oxygen concentration and low oxygen concentration occur, the ventilator parameters adjustment instruction carries the information about the abnormal events that occur

S7

While receiving the mechanical ventilation therapy data in real time, streaming media data of the treatment scenes is also received in real time; live broadcast in real time of the waveform data, the numeric data, and the treatment scenes in the mechanical ventilation therapy data of the medical data set as well as the mechanical ventilation therapy data analysis reports of some or all patients in one or more wards

FIG. 1

**Mechanical Ventilation Data Object**

PEEP: float
Fspn: int
Volume: byte[ ]
...

202

**Physiological Parameters Data Object**

HR: int
Temp: int
ECG: byte[ ]
...

203

**Blood Gas Analysis Data Object**

Ph: float
HCO3-: float
SaO2: float
...

204

**Laboratory Diagnostic Data Object**

PLT: float
MCV: float
blood_type: int
...

205

**Standard Data Structure Object**

201

**Image Data Object**

ID: int
Type: int
imageID: int
...

206

**Clinical Diagnosis And Treatment Information Object**

Ph: float
CHOL: float
imageID: int
...

207

**Patient Information Object**

PID: string
Name: string
Age: int
...

208

**Metadata Information Object**

ID: int
Time: timestamp
Ver: int
Flag: boolean
...

209

**Extension Object 1**

.......

**Extension Object N**

FIG. 2

$T$       $T'$       $T''$

$S_1$

$S_2$   $b = 4$   $G_1$

$S_3$                  $f = 2$   $H_1$

$S_4$   $b = 4$   $G_2$

$b = 4$   $G_{C'}$       $f = 2$   $H_{C''}$

FIG. 3 (a)

$T'$                            $T''$

$NOR_{c\_Mean}$

$NOR_{c\_Max}$

$NOR_{c\_Min}$

$NOR_{c\_25Q}$

$NOR_{c\_50Q}$

$NOR_{c\_75Q}$

$NOR_{c\_Std}$

$NOR_{c\_p2p}$

$NOR_c$            $DON_{c\_Mean}$

$DON_c$           $DON_{c\_Max}$

$DON_{c\_Min}$

$DON_{c\_25Q}$

$DON_{c\_50Q}$

$DON_{c\_75Q}$

$DON_{c\_Std}$

$DON_{c\_p2p}$

$G_c$    $2*3$                $H_c$    $16*3$

FIG. 3 (b)

## XXXX hospital

Report No. 9082116540333-1    **Mechanical ventilation therapy data analysis report**

| Patient Name | Li*Min | Gender | Male | Age | 63 Years old |
|---|---|---|---|---|---|
| Height | 175 cm | Body Weight | 90 kg | Blood Type | A, Rh- |
| Hospital Departments | Critical Care Medicine Center | Ward | Surgical Intensive Care Unit | Bed | 04 |
| Reporting Time | 2022-04-28 20:54:28 | Service Code | 019082116540333 | Hospital Number | 223456 |
| Clinical Diagnosis | Acute respiratory distress syndrome | | | | |
| Data Start Time | 2022-04-27 14:54:28 | Data end Time | 2022-04-28 14:54:28 | Data Duration | 24:00:00 |
| Ventilator Device ID | MA3872903 | Monitor Device ID | MA93749274 | Intubation Type | Tracheotomy |
| Mechanical Ventilation Mode | P-SIMV | Boot Time | 2022-04-22 16:54:28 | Weaning Time | -- |

### Breath Analysis

| | | | |
|---|---|---|---|
| Total Respiratory Cycle | 8800 | Spontaneous Breathing Cycle | 6800 |
| Spontaneous breathing frequency ratio | 77% | Shallow and rapid breathing index | 111 times/min/L |

### Patient-ventilator async (PVA) Analysis

| | | | |
|---|---|---|---|
| PVA Cycles | 2725 Times | Async Index | 28% |
| Ineffective Effort | 1000 Times (10%) | Double-Triggering | 201 Times (2%) |
| Auto-Triggering | 300 Times (3%) | Reverse-triggering | 290 Times (3%) |
| Flow Starvation | 209 Times (2%) | Overshoot | 365 Times (4%) |
| Delayed Cycling | 160 Times (2%) | Premature Cycling | 200 Times (2%) |

### Analysis Of Changes In Airway Resistance

| | | | |
|---|---|---|---|
| Increased Airway Resistance Events | 12 Times | Cumulative Duration Of Increased Airway Resistance | 02:30:25 |

The longest Duration Of Increased Airway Resistance: 28 Minutes, Occurred On 04-27 23:07:11

### Compliance Change Analysis

| | | | |
|---|---|---|---|
| Compliance Reduction Events | 9 Times | Cumulative Duration Of Reduced Compliance | 01:41:17 |

The longest Duration Of Reduced Compliance: 31 Minutes, Occurred On 04-28 04:07:37

### Lung Injury Early Warning Analysis

| | | | |
|---|---|---|---|
| Lung Damage Warning | 6 Times | Time Of Most Severe Lung Injury | 2022-04-28 06:14:32 |
| Decreased Systolic Blood Pressure (<100 mmHg) | 99 mmHg | Decreased Oxygen Saturation (<85%) | 80% |
| Decreased Compliance (<25 mL/cmH$_2$O) | 24 mL/cmH$_2$O | Decreased Diastolic Blood Pressure (<50 mmHg) | 40 mmHg |
| Plateau Pressure Rises (>30cmH$_2$0) | 36 cmH$_2$0 | Progressive Increase In Peak Airway Aressure (>35 cmH$_2$0) | 50 cmH$_2$O |

1. Mechanical ventilation monitoring: 24 hours;

2. Total respiratory cycles: 8800, spontaneous respiratory cycles: 6800, spontaneous respiratory rate ratio: 77%, shallow rapid breathing index: 111 times/min/L.

3. Total number of PVA cycles: 2725, asynchrony index: 28%, among which "Ineffective effort, auto-triggering, overshoot" are the main PVA asynchrony events.

4. Events of increased airway resistance: 12 times, the longest duration: 28 minutes, occurred on 2022-04-27 23:07:11.

5. Compliance reduction events: 9 times, the longest duration: 31 minutes, occurred at 2022-04-28 04:07:37.

6. Lung injury risk warning: 6 times, the most serious lung injury occurred on 2022-04-28 06:14:32.

Diagnosing Physician (SignVature):

| 9 Pages In Total | This report is for clinical reference only | Page 1 |
|---|---|---|

FIG. 4

| Name | Li*min | Gender | male | Age 63 Years old | | Hospital Number | 223456 |

**501** — Peak Max 20 Occurred on 04-28 00:51:53 Min 17 Occurred on 04-27 20:00:13; PEEP Max 11 Occurred on 04-27 23:41:29 Min 9 Occurred on 04-27 16:07:39 (Ppeak, PEEP)

**502** — VTi Max 800 Occurred on 04-27 04:44:31 Min 9 Occurred on 04-28 08:30:57; VTe Max 850 Occurred on 04-28 04:50:15 Min 9 Occurred on 04-28 08:47:23 (VTi, VTe)

**503** — MVi Max 13.2 Occurred on 04-27 19:50:32 Min 13.0 Occurred on 04-28 04:13:01; MVe Max 9.9 Occurred on 04-27 19:20:20 Min 7.0 Occurred on 04-28 03:59:23 (MVi, MVe)

**504** — Ftot Max 40 Occurred on 04-27 19:50:31 Min 20 Occurred on 04-28 04:13:01; Fspon Max 31 Occurred on 04-27 19:20:18 Min 0 Occurred on 04-28 03:59:23 (ftot, fspon)

**505** — FiO2 Max 43 Occurred on 04-27 20:00:23 Min 39 Occurred on 04-27 16:07:18

**506** — Tips And Discoveries

1. The trend is stable, with no obvious abnormalities

**500**

FIG. 5

**FIG. 6(b)**

| Name Li*min | | Gender male | Age 63 years old | | Hospital number 223456 |
|---|---|---|---|---|---|

Mechanical ventilation monitoring parameter record sheet

| Parameter | Unit | Maximum value | | Minimum value | | Remark |
|---|---|---|---|---|---|---|
| Ppeak | cmH20 | 43 | Occurred on 04-27 02:26:28 | 21 | Occurred on 04-27 01:26:28 | |
| Pplat | cmH20 | 27 | Occurred on 04-27 05:11:01 | 18 | Occurred on 04-27 05:11:01 | |
| Pmean | cmH20 | 32 | Occurred on 04-28 03:20:09 | 21 | Occurred on 04-28 13:20:09 | |
| PEEP | cmH20 | 16 | Occurred on 04-27 03:06:18 | 12 | Occurred on 04-27 23:06:18 | |
| MVi | mL | 800 | Occurred on 04-27 05:20:13 | 480 | Occurred on 04-27 19:20:13 | |
| MVe | mL | 850 | Occurred on 04-27 10:20:08 | 762 | Occurred on 04-27 20:20:08 | |
| TVe spn | mL | 660 | Occurred on 04-28 07:04:42 | 430 | Occurred on 04-28 04:04:42 | |
| TVe /IBW | mL/Kg | 650 | Occurred on 04-28 13:54:00 | 520 | Occurred on 04-28 11:54:00 | |
| MVspn | L/min | 13.2 | Occurred on 04-28 13:54:00 | 13.0 | Occurred on 04-27 21:51:18 | |
| MVleak | L/min | 9.9 | Occurred on 04-27 10:31:20 | 7.0 | Occurred on 04-27 18:31:20 | |
| Ftotal | bpm | 29 | Occurred on 04-27 02:26:28 | 19 | Occurred on 04-27 22:26:28 | |
| Fmand | bpm | 20 | Occurred on 04-28 10:20:08 | 10 | Occurred on 04-28 01:20:08 | |
| Fspon | bpm | 29 | Occurred on 04-28 07:04:42 | 9 | Occurred on 04-27 17:04:42 | |
| Ri | cmH20/(L/s) | – | Occurred in | – | Occurred in | |
| Re | cmH20/(L/s) | – | Occurred in | – | Occurred in | |
| Cstat | ml/cmH20 | 180 | Occurred on 04-27 10:31:20 | 160 | Occurred on 04-27 20:31:20 | |
| Cdyn | ml/cmH20 | 150 | Occurred on 04-27 02:26:28 | 130 | Occurred on 04-27 22:26:28 | |
| RSBI | 1/(min·L) | 21 | Occurred on 04-28 10:20:08 | 13 | Occurred on 04-28 04:20:08 | |
| WOB | J/min | – | Occurred in | – | Occurred in | |
| Leak% | % | 32 | Occurred on 04-27 10:31:20 | 32 | Occurred on 04-27 21:31:20 | |
| RCexp | s | 16 | Occurred on 04-27 02:26:28 | 16 | Occurred on 04-27 02:26:28 | |
| NIF | cmH20 | 20 | Occurred on 04-28 10:20:08 | 20 | Occurred on 04-28 02:20:08 | |
| P0.1 | cmH20 | 21 | Occurred on 04-28 23:51:18 | 21 | Occurred on 04-28 03:15:18 | |
| PEEPi | cmH20 | 28 | Occurred on 04-27 05:11:01 | 13 | Occurred on 04-27 22:11:01 | |
| Vtrap | mL | 21 | Occurred on 04-28 03:20:08 | 21 | Occurred on 04-28 12:20:09 | |
| FI02 | % | 40 | Occurred on 04-28 03:06:18 | 33 | Occurred on 04-27 18:06:18 | |
| EtC02 | mL | 42 | Occurred on 04-28 05:20:13 | 19 | Occurred on 04-27 21:20:13 | |
| VDaw/Tve | – | – | Occurred in | – | Occurred in | |
| Vtalv | mL | – | Occurred in | – | Occurred in | |
| V'alv | mL/min | – | Occurred in | – | Occurred in | |
| slopeC02 | – | – | Occurred in | – | Occurred in | |
| V'C02 | bpm | – | Occurred in | – | Occurred in | |
| VeC02 | mL | – | Occurred in | – | Occurred in | |
| ViC02 | mL | – | Occurred in | – | Occurred in | |

**FIG. 6(a)**

| Name Li*min | | Gender male | Age 63 years old | | Hospital number 223456 |
|---|---|---|---|---|---|

Mechanical ventilation monitoring parameter record sheet

| Ventilation mode / Record time | | V-A/C 00:26:00 | SIMV+PSV 06:26:00 | PRVC-SIMV 12:26:00 | P-A/C 18:26:00 | CPAP/PSV 00:25:59 |
|---|---|---|---|---|---|---|
| TV | mL | 480 | 450 | 450 | – | – |
| O2 | % | 30 | 40 | 50 | 45 | 40 |
| I:E | – | 1:2 | 1:2 | 1:2 | 1:2 | – |
| PEEP | cmH20 | 3 | 3 | 3 | 3 | 3 |
| f | bpm | 20 | 20 | 20 | 20 | – |
| fsimv | bpm | 20 | 20 | 20 | 20 | – |
| Tslop | s | – | – | – | – | 0.20 |
| ΔPsupp | cmH20 | – | – | – | – | 0 |
| ΔPinsp | cmH20 | – | – | – | – | – |
| Tinsp | s | 1s | 1s | 1s | 1s | – |
| Tpause | % | 8 | 8 | 8 | 8 | – |
| F-Trig | L/min | 2.0 | 2.0 | 2.0 | 2.0 | 2.0 |
| P-Trig | cmH20 | – | – | – | – | – |
| Exp% | % | – | 25 | 25 | – | Auto |
| Phigh | cmH20 | – | – | – | – | – |
| Plow | cmH20 | – | – | – | – | – |
| Thigh | s | – | – | – | – | – |
| Tlow | s | – | – | – | – | – |
| Apnea Tinsp | s | 30 | 30 | 30 | 30 | 30 |
| TVapnea | mL | – | – | – | – | – |
| ΔPapnea | cmH20 | 15 | 15 | 15 | 15 | 15 |
| Fapnea | bpm | 10 | 10 | 10 | 10 | 10 |
| Apnea Tinsp | s | 1 | 1 | 1 | 1 | 1 |

Ventilation mode and setting parameter change diagram

Legend: Total respiratory rate (bpm) — Spontaneous respiratory rate (bpm) — Positive end-expiratory pressure (cmH2O)

## FIG. 6(d) — 641

| Name Li*min | Gender male | Age 63 years old | Hospital number 223456 |
|---|---|---|---|

**24-hour blood gas analysis monitoring record**

| time | pH | PaO2 mmHg | PaCO2 mmHg | HCO3 mmol/L | BE mmol/L | FIO2 % | PEEP mmHg | Remark |
|---|---|---|---|---|---|---|---|---|
| 15:00 | 7.401 | 85 | 39.7 | 24.1 | 3.0 | 30 | 40 | |
| 19:00 | 7.392 | 89 | 38.3 | 24.2 | 1.0 | 21 | 41 | |
| 23:00 | 7.408 | 83 | 40.5 | 23.5 | 3.0 | 45 | 39 | |
| 03:00 | 7.412 | 89 | 39.0 | 24.7 | 2.0 | 51 | 37 | |
| 07:00 | 7.291 | 90 | 38.8 | 24.2 | 2.0 | 46 | 45 | |
| 11:00 | 7.312 | 91 | 35.6 | 27.4 | 3.0 | 34 | 45 | |
| 15:00 | 7.271 | 89 | 39.1 | 24.6 | 0.9 | 45 | 41 | |

642

PaO2/PaCO2/HCO3 Trend Chart

PaO2 (mmHg) / PaCO2 (mmHg) ─○─ PaO2 ─✱─ PaCO2 ─◇─ HCO3   HCO3 (mmol/L)

643

**Experimental diagnostic data record form**

Test content: blood routine
Inspection time:2022-04-27 20:00:00

| Inspection items | | Result | Reference range |
|---|---|---|---|
| Total number of white blood cells | WBC | 5.26 x 10^9/L | 4.00 ~ 10.00 |
| Number of neutrophils | GRAN | 4.94 x 10^9/L | 2.00 ~ 7.00 |
| Number of red blood cells | RBC | 3.94 x 10^9/L | 3.50 ~ 5.00 |
| Hemoglobin concentration | HGB | 99g/L | 110 ~ 150 |
| Platelet concentration | PLT | 166 x 10^9/L | 100 ~ 300 |

Clinical diagnosis: low hemoglobin concentration

**FIG. 6(d)**

## FIG. 6(c) — 631 / 640 / 632 / 630

| Name Li*min | Gender male | Age 63 years old | Hospital number 223456 |
|---|---|---|---|

**24-hour vital signs monitoring record**

| Time | Heart rate (bpm) Maximum | Minimum | Average | Respiration rate (rpm) Maximum | Minimum | Average | Blood oxygen saturation (%) Maximum | Minimum | Average | Invasive arterial pressure (mmHg) Systolic | diastolic | mean pressure |
|---|---|---|---|---|---|---|---|---|---|---|---|---|
| 15:00 | 87 | 35 | 69 | 26 | 13 | 20 | 100 | 80 | 97 | 129 | 61 | 93 |
| 16:00 | 98 | 33 | 66 | 26 | 13 | 20 | 100 | 98 | 99 | 123 | 44 | 90 |
| 17:00 | 98 | 45 | 83 | 26 | 13 | 20 | 100 | 80 | 97 | 133 | 64 | 87 |
| 18:00 | 117 | 45 | 83 | 26 | 13 | 20 | 100 | 98 | 99 | 140 | 59 | 90 |
| 19:00 | 121 | 61 | 81 | 26 | 13 | 20 | 100 | 80 | 97 | 128 | 59 | 90 |
| 20:00 | 87 | 35 | 69 | 26 | 13 | 20 | 100 | 98 | 99 | 153 | 71 | 104 |
| 21:00 | 98 | 33 | 66 | 26 | 13 | 20 | 100 | 80 | 97 | 129 | 61 | 93 |
| 22:00 | 98 | 45 | 83 | 26 | 13 | 20 | 100 | 98 | 99 | 123 | 44 | 90 |
| 23:00 | 117 | 45 | 83 | 26 | 13 | 20 | 100 | 80 | 97 | 133 | 64 | 87 |
| 00:00 | 121 | 61 | 81 | 26 | 13 | 20 | 100 | 98 | 99 | 140 | 59 | 90 |
| 01:00 | 87 | 35 | 69 | 26 | 13 | 20 | 100 | 80 | 97 | 128 | 59 | 90 |
| 02:00 | 98 | 33 | 66 | 26 | 13 | 20 | 100 | 98 | 99 | 153 | 71 | 104 |
| 03:00 | 98 | 45 | 83 | 26 | 13 | 20 | 100 | 80 | 97 | 129 | 61 | 93 |
| 04:00 | 117 | 45 | 83 | 26 | 13 | 20 | 100 | 98 | 99 | 123 | 44 | 90 |
| 05:00 | 121 | 61 | 81 | 26 | 13 | 20 | 100 | 80 | 97 | 133 | 64 | 87 |
| 06:00 | 87 | 35 | 69 | 26 | 13 | 20 | 100 | 98 | 99 | 140 | 59 | 90 |
| 07:00 | 98 | 33 | 66 | 26 | 13 | 20 | 100 | 80 | 97 | 128 | 59 | 90 |
| 08:00 | 98 | 45 | 83 | 26 | 13 | 20 | 100 | 98 | 99 | 153 | 71 | 104 |
| 09:00 | 117 | 45 | 83 | 26 | 13 | 20 | 100 | 80 | 97 | 129 | 61 | 93 |
| 10:00 | 121 | 61 | 81 | 26 | 13 | 20 | 100 | 98 | 99 | 123 | 44 | 90 |
| 11:00 | 87 | 35 | 69 | 26 | 13 | 20 | 100 | 80 | 97 | 133 | 64 | 87 |
| 12:00 | 98 | 33 | 66 | 26 | 13 | 20 | 100 | 98 | 99 | 140 | 59 | 90 |
| 13:00 | 98 | 45 | 83 | 26 | 13 | 20 | 100 | 80 | 97 | 128 | 59 | 90 |
| 14:00 | 117 | 45 | 83 | 26 | 13 | 20 | 100 | 98 | 99 | 153 | 71 | 104 |

Heart rate/respiration rate/blood pressure trend graph

Invasive arterial pressure (mmHg)/heart rate (bpm)          Respiration rate (rpm)

systolic blood pressure
diastolic blood pressure
respiratory rate
heart rate

**FIG. 6(c)**

## FIG. 6(f)

### Imaging examination report

#### Ultrasound images

Upper right blue dot | lower right blue dot | right diaphragm point
upper left blue dot | lower left blue dot | left diaphragm point

Tips and findings: Ultrasound showed lesions in the right middle and lower lungs, manifested as interstitial lung syndrome, fragmentation sign and consolidation, and a small amount of pleural effusion. Subpleural lesions and a small amount of pleural effusion were seen in the left lower lung, and the lung ultrasound signs were consistent with ARDS.

#### CT image

Tips and findings: There are multiple patches and cord shadows in the multi-lobe segments of both lungs. Ground glass density shadow, partial consolidation, mainly in the right lung, considered inflammatory changes, increased compared with the previous lesions. Signs of emphysema in both lungs.

#### DR image

Tips and discoveries:

The translucency of both lungs is reduced, the texture of both lungs is slightly increased and blurred, and flocculent slightly high-density shadows are seen, with a little inflammation or other changes;

There are patchy high-density shadows in the middle and lower fields of the right lung, and the right diaphragmatic surface is not clear. Please combine clinical and related examinations; the heart shadow is not large, and the right heart edge is not clear; the aortic node is calcified; there is a small amount of effusion in the right pleural cavity; the right costophrenic angle is sharp.

The quality of bedside radiography is limited. It is recommended that the department review or combine it with relevant examinations.

## FIG. 6(e)

### Alarm monitoring record form

Total alarm data     42 physiological alarm data     40 technical alarm data

| Event name | Maximum value | | Minimum value | | Alarm frequency Second-rate | Proportion % |
|---|---|---|---|---|---|---|
| airway pressure too high | 25 | Occurred on 04-28 02:26:28 | 23 | Occurred on 04-27 20:16:28 | 9 | 21 |
| airway pressure is too low | 18 | Occurred on 04-27 05:26:28 | 8 | Occurred on 04-27 23:16:28 | 2 | 4 |
| FiO2 is too high | 6 | Occurred on 04-28 03:26:30 | 5 | Occurred on 04-28 19:16:28 | 2 | 4 |
| FiO2 is too low | 4 | Occurred on 04-27 04:26:10 | 3 | Occurred on 04-28 18:16:28 | 3 | 7 |
| Oxygen concentration too high | .. | | .. | | .. | .. |
| Oxygen concentration too low | .. | | .. | | .. | .. |
| Breathing rate is too high | 90 | Occurred on 04-28 12:00:31 | 89 | Occurred on 04-28 04:34:01 | 1 | 2 |
| Breathing rate too low | 50 | Occurred on 04-28 09:01:22 | 49 | Occurred on 04-28 02:20:02 | 1 | 2 |
| Exhaled tidal volume is too high | 598 | Occurred on 04-28 07:26:28 | 580 | Occurred on 04-28 15:16:28 | 4 | 9 |
| Exhaled tidal volume is too low | 340 | Occurred on 04-28 08:26:28 | 333 | Occurred on 04-28 14:16:28 | 1 | 2 |
| Minute ventilation is too high | 11 | Occurred on 04-28 09:26:28 | 10 | Occurred on 04-28 13:16:28 | 3 | 7 |
| Minute ventilation too low | 4 | Occurred on 04-28 10:26:28 | 3 | Occurred on 04-28 12:16:28 | 2 | 4 |
| High PEEP | 110 | Occurred on 04-27 18:26:28 | 108 | Occurred on 04-28 05:16:28 | 2 | 4 |
| Low PEEP | 5 | Occurred on 04-27 19:26:28 | 4 | Occurred on 04-28 04:16:28 | 1 | 2 |
| Et CO2 too high | 133 | Occurred on 04-27 17:26:28 | 133 | Occurred on 04-28 08:16:28 | 3 | 7 |
| Et CO2 too low | 133 | Occurred on 04-27 16:26:28 | 133 | Occurred on 04-28 07:16:28 | 8 | 19 |
| Asphyxia | .. | | .. | | .. | .. |
| Apnea ventilation | .. | | .. | | .. | .. |
| End of apnea ventilation | .. | | .. | | .. | .. |

#### 24-hour alarm event distribution map

### Tips and discoveries

1. Excessive airway secretions and drainage obstruction, loss of airway protection function

2. Use volume control mode with an inspiratory time > 0.4 seconds and leak compensation enabled.

3. The patient produces strong spontaneous breathing with a sustained expiratory flow rate of >90 L/min and a duration of >0.4 seconds and the difference between the set positive end-expiratory pressure and the high pressure alarm isValue <35cmH2O

4. Increased airway resistance and lung compliance, accompanied by significant airway obstruction, prolonged time constant, leading to gas trapping

FIG. 7

FIG. 8

FIG. 9

FIG. 10

FIG. 11

Report Generation Unit

Stream Media Management Unit

1210

1204

Data Cleaning Unit | Data Labeling Unit

Data Acquisition And Standardization Unit

Edge Computing Operating System

1211

1202

1203

1201

1209

1208

1207

1205

1209

1207

1206

1205

1208

FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020230336 A1
- CN 113577476 A
- US 2021074415 A1
- US 10874811 B2
- EP 2249700 B1